(19) Europäisches Patentamt
European Patent Office
Office européen des brevets

(11) **EP 1 176 949 B1**

(12) **EUROPÄISCHE PATENTSCHRIFT**

(45) Veröffentlichungstag und Bekanntmachung des Hinweises auf die Patenterteilung:
**10.12.2014 Patentblatt 2014/50**

(51) Int Cl.:
***A61K 9/16*** *(2006.01)*     ***A61K 9/50*** *(2006.01)*

(21) Anmeldenummer: **00931138.2**

(86) Internationale Anmeldenummer:
**PCT/EP2000/004112**

(22) Anmeldetag: **08.05.2000**

(87) Internationale Veröffentlichungsnummer:
**WO 2000/067728 (16.11.2000 Gazette 2000/46)**

(54) **LIPIDPARTIKEL AUF DER BASIS VON MISCHUNGEN VON FLÜSSIGEN UND FESTEN LIPIDEN UND VERFAHREN ZU IHRER HERSTELLUNG**

LIPID PARTICLES ON THE BASIS OF MIXTURES OF LIQUID AND SOLID LIPIDS AND METHOD FOR PRODUCING SAME

PARTICULES LIPIDIQUES A BASE DE MELANGES DE LIPIDES LIQUIDES ET SOLIDES ET PROCEDE POUR LES PRODUIRE

(84) Benannte Vertragsstaaten:
**AT BE CH CY DE DK ES FI FR GB GR IE IT LI LU MC NL PT SE**
Benannte Erstreckungsstaaten:
**SI**

(30) Priorität: **07.05.1999 DE 19921034**
**09.08.1999 DE 19938371**
**21.09.1999 DE 19945203**
**31.03.2000 DE 10016357**

(43) Veröffentlichungstag der Anmeldung:
**06.02.2002 Patentblatt 2002/06**

(73) Patentinhaber: **PharmaSol GmbH**
**12307 Berlin (DE)**

(72) Erfinder:
• **MÜLLER, Rainer, Helmut**
**D-12161 Berlin (DE)**
• **JENNING, Volkhard**
**D-10551 Berlin (DE)**
• **MÄDER, Karsten**
**D-13187 Berlin (DE)**
• **LIPPACHER, Andreas**
**D-12159 Berlin (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 448 930     EP-A- 0 525 307**
**US-A- 5 250 236**

## Beschreibung

Lipidpartikel unterschiedlicher Größe finden Einsatz zur kontrollierten Arzneistoffapplikation. Arzneistoffbeladene Lipidpellets mit einer Größe von ca. 0,10 - 2,0 mm werden in Hartgelatinekapseln gefüllt, aus den Lipidpellets wird der Arzneistoff retardiert freigesetzt (Handelspräparat Mucosolvan®, [Müller, R.H., Feste Lipidnanopartikel (SLN), in Müller, R.H., Hildebrand, G.E. (Hrsg), Pharmazeutische Technologie: Moderne Arzneiformen, Wissenschaftliche Verlagsgesellschaft Stuttgart, 357 - 366, 1998]). Lipidmikropartikel können für unterschiedliche Applikationswege eingesetzt werden, von topischen Produkten (z.B. O/W-Cremes) über orale Arzneimittel bis hin zu Parenteralia. Eine Größenklasse kleiner sind die festen Lipidnanopartikel (Solid Lipid Nanoparticles - SLN®), die ein noch breiteres Einsatzgebiet besitzen. Aufgrund der Feinheit der Partikelgröße ist z.B. auch eine ophthalmologische Anwendung möglich.

Lipidpartikel können in Form einer fließfähigen Dispersion angewendet werden, d.h. die Lipidpartikel sind dispergiert in einer wäßrigen Phase (z.B. in isotonischer Glukoselösung) oder in einer nicht-wäßrigen Phase (z.B. in PEG 600 oder Öl). Bei Anwendung als Dispersion muß das System in der Regel fließfähig sein, d.h. von niedriger Viskosität. Die Lipidkonzentration in den Dispersionen ist in der Regel relativ niedrig im Bereich von ca. 1 - 10 % (Gewichtsprozente) . Für die meisten Anwendungszwecke ist dies ausreichend. Falls notwendig, kann die Lipidpartikelkonzentration auch ohne größere Probleme auf bis zu 20 % erhöht werden (analog 20%igen Emulsionen zur parenteralen Ernährung).

Die Situation bezüglich der erforderlichen Lipidkonzentration ist anders bei der Einarbeitung von Lipidpartikeln in traditionelle Arzneiformen wie Cremes, orale Arzneiformen wie Tabletten, Pellets und Kapseln sowie bei Parenteralia mit beschränktem Injektionsvolumen. Hier sind wesentlich höhere Lipidkonzentrationen erforderlich um den Wasseranteil der Dispersion zu reduzieren, der z.B. zur Herstellung der Tablette entfernt werden muß. Dies gilt insbesondere bei hohen einzuarbeitenden Lipidmengen in diese Arzneiformen, welche durch niedrige Arzneistoffbeladungskapazität der Partikel bedingt sind.

Die Einarbeitung hochkonzentrierter Lipidpartikel in diese Arzneiformen ist unproblematisch, wenn es sich um relativ große Partikel handelt (> 100 $\mu$m). Die Lipide können als grobes Pulver mit einer konventionellen Mühle gemahlen werden. Die so erhaltenen Partikel in einer Größe von 100 - 200 $\mu$m werden als Pulver im Herstellungsprozeß zur Arzneiform zugemischt.

Schwieriger ist die Situation bei Lipid-Mikropartikeln und Lipidnanopartikeln. Bei Lipidmikropartikeln im Bereich von ca. 1 - 100 $\mu$m ist ein hochenergetisches Mahlen erforderlich, um diese Feinheit der Partikelgröße zu erreichen. Die zwangsläufig auftretende Wärme beim Mahlprozeß kann zu Anschmelzen des Lipids und Verklumpungen führen, Gegenkühlung ist in der Regel notwendig. Feinteilige Pulver, speziell bei hydrophober Oberfläche, neigen zur Partikelaggregation. Um diese Probleme zu vermeiden, ist die Naßmahlung essentiell, gegebenenfalls unter Zusatz eines Tensides. Hochfeine Lipidpartikel, d.h. im Bereich weniger Mikrometer und insbesondere im Nanometerbereich, können durch Trockenmahlung in der Regel nicht erzeugt werden. Zur Naßmahlung wird das grobe Lipidpulver in einer Flüssigkeit dispergiert und mit einer entsprechenden Flüssigkeitsmühle bearbeitet (z.B. Kolloidmühle) . Weitere Herstellmöglichkeiten sind Hochdruckhomogenisationsverfahren [Müller, R.H., Feste Lipidnanopartikel (SLN), in Müller, R.H., Hildebrand, G.E. (Hrsg), Pharmazeutische Technologie: Moderne Arzneiformen, Wissenschaftliche Verlagsgesellschaft Stuttgart, 357 - 366, 1998] oder alternativ Präzipitation [Morel, S., Ugazio, E., Cavalli, R., Gasco, M.R., Thymopentin in solid lipid nanoparticles, Int. J. Pharm., 259 - 261, 1996]. Hochfeine Lipidpartikel können in der Regel nur in Form einer Dispersion in die oben genannten Arzneiformen eingearbeitet werden.

Hochkonzentrierte Lipidpartikel-Dispersionen sind essentiell zur Herstellung von oralen Arzneiformen und bestimmten Parenteralia. So wird bei der Herstellung von Tabletten die wäßrige Lipidpartikeldispersion als Granulierflüssigkeit eingesetzt. Die zur Einarbeitung einer bestimmten Lipidpartikelmenge als Granulierflüssigkeit zu verwendenden Volumina an wäßriger Lipidpartikeldispersion dürfen nicht zu hoch sein, da sonst zu viel Wasser entfernt werden muß bzw. eine Granulierung gar nicht mehr möglich ist. Analoges gilt für die Verwendung von wäßrigen Lipidpartikeldispersionen zum Anteigen der Hilfsstoffmischung zur Pelletextrusion. Weichgelatinekapseln können mit nicht-wäßrigen Lipidpartikeldispersionen gefüllt werden. Um bei einer gegebenen maximalen Arzneistoffbeladungskapazität des Lipids das maximal mögliche Füllvolumen nicht zu überschreiten, muß auch hier die Lipidpartikeldispersion ausreichend hoch an Lipidpartikeln konzentriert sein.

Dies sei an einem Beispiel erläutert. Die Einzeldosis von Cyclosporin beim Erwachsenen beträgt ca. 200 mg. Die unter Verwendung von Cyclosporin hergestellten Lipidnanopartikel haben eine maximale Beladungskapazität von 20 %, d.h. die Lipidmatrix besteht aus 200 mg Cyclosporin und 800 mg Lipid [Müller, R.H., Runge, S.A., Ravelli, V., Pharmazeutische Ciclosporin-Formulierung mit verbesserten bio-pharmazeutischen Eigenschaften, erhöhter physikalische Qualität und Stabilität sowie ein Verfahren zur Herstellung derselben, DE 198 19 273]. Diese Einzeldosis soll in zwei Tabletten à 1 g verabreicht werden, d.h. 1 g Lipid-Cyclosporin-Partikel muß mit 1 g Hilfsstoffen zur Tablettierung im Granulierungsprozeß angeteigt werden. Bei dem momentanen Stand der Lipidnanopartikel-Herstellungstechnologie sind 1 g Cyclosporin-beladene Lipidnanopartikel in 4 g Wasser dispergiert (Gesamtvolumen der wäßrigen Lipidnanopartikeldispersion: ca. 5 ml = 5 g). Bei Zumischung dieser 5 g zu 1 g Tablettenhilfsstoffen müssen somit 4 g Wasser entfernt werden, nach Wasserentfernung erhält man 2 g Tablettenmischung. Es ist offensichtlich, daß bei derartig niedrig konzentrierten Lipidnanopartikeldispersionen eine Granulierung nicht möglich ist (Entfernung von 4 g Wasser aus 6 g Granulatmischung). Notwendig sind Lipidpartikeldispersionen mit einem Lipidgehalt von 50-70 %. Ähnliche Probleme ergeben sich bei a) Arzneistoffen mit durchschnittlich hoher Einmaldosis bei Einarbeitung von Arzneistoff-beladenen Lipidpartikeln in sämtliche traditionelle Arzneiformen und b) Arzneistoffen, die zwar eine geringe Einmaldosis haben, sich jedoch schlecht in Lipidpartikel einarbeiten lassen (= niedrige Beladungskapazität).

Gemäß EP 0 448 930 und EP 0 525 307 werden durch Schmelzmischen von Fett und/oder Wachs sowie biologisch aktivem Protein, Peptid oder Polypeptid und Zumischen von 1 bis 30% eines Öls, halbfesten Fetts und/oder Fettsäurederivats mit anschließender Sprühkristallisation Partikel gebildet, deren Strukturen aufgrund von einem geringen Anteil an Öl, halbfestem Fett, und/oder Fettsäurederivat sehr schnell aus der flüssigen $\alpha$-Struktur der Schmelze in die stabile feste kristalline ß-Form umgewandelt werden.

Ausgehend davon hat der vorliegenden Erfindung die Aufgabe zugrunde gelegen, Lipidpartikeldispersionen bereitzustellen, die neben der Möglichkeit in Bezug auf den Lipidanteil hochkonzentriert zu sein auch die Möglichkeit einer hohen Wirkstoffbeladungskapazität und einer verbesserten Stabilität in Bezug auf Agglomeratbildung bieten.

Diese Aufgabe wird erfindungsgemäß durch eine Lipidpartikeldispersion gelöst, die dadurch gekennzeichnet ist, daß sie Lipidpartikel umfasst, die eine gemischte Matrix aus festem Lipid und flüssigem Lipid aufweisen, integer sind und eine innere Struktur aufweisen, die sich durch eine nicht vollständige Kristallinität in der ß-Modifikation auszeichnet, wie bestimmt durch Differential Scanning Calorimetry (DSC) und Röntgendiffraktometrie, wobei das flüssige Lipid, oder eine Mischung aus flüssigen Lipiden, und das feste Lipid, oder eine Mischung aus festen Lipiden in einem Verhältnis von 50 + 50 bis 0,1 + 99,9 oder 99 + 1 bis 50 + 50 gemischt werden, die Lipidpartikel in einer äußeren Phase (Dispersionsmedium) dispergiert vorliegen (Dispersion), wobei die Lipide durch Anwendung von Hockdruckhomogenisation dispergiert werden, wobei der Zusatz an Lipidphase zu dem Dispersionsmedium in einem Schritt oder sukzessive in Teilschritten erfolgt, die Lipide liegen dabei oberhalb ihres Schmelzpunktes im flüssigen Zustand oder im festen und/oder teilweise festen Zustand vor, und die Dispersion zusammengesetzt ist aus:

a) einem oder mehreren Ölen als Lipidpartikelbestandteil, welche bei 4 °C flüssig sind,

b) einem oder mehreren lipophilen Stoffen als Lipidpartikelbestandteil, die bei 37 °C fest sind und das flüssige Öl verfestigen,

c) einer Wasserphase oder mit Wasser mischbaren Phase als Dispersionsmedium,

d) einem oder mehreren Stoffen zur Erhöhung der physikalischen Stabilität der Dispersion,

e) keinem, einem oder mehreren Wirkstoffen, die sich überwiegend in den Lipidtröpfchen befinden.

Die Herstellung von Lipidmikropartikeldispersionen im unteren Mikrometerbereich wird in mehreren Patenten, Patentanmeldungen und in der Literatur beschrieben. Die maximal eingesetzten Lipidkonzentrationen laut Patentansprüchen bzw. Beispielen sind dabei z.B. 3 % [Domb, A., Lipospheres for controlled delivery of substances, US-A-5,188,837, 1993], 30 % [Gasco, M.R., Method for producing solid lipid micro-spheres having a narrow size distribution, US-A-5,250,236, 1993] und 20 % [Speiser, P., Lipidnanopellets als Trägersystem für Arzneimittel zur peroralen Anwendung, Europäisches Patent EP 0 167 825, 1990]. Bei höheren Konzentrationen wird die Bildung von Gelen (Fett in Wasser) oder Salben beschrieben (Wasser in Fett dispergiert). Die maximal eingesetzten Fettmengen zur Herstellung von Lipidnanopartikeln betragen 30 % [Müller, R.H., Lucks, J.S., Arzneistoffträger aus festen Lipidteilchen, Solid Lipid Nanospheres (SLN), Europäisches Patent EP 0 605 497, 1996] Auch bei den Lipidnanopartikeln wird bei Verwendung höherer Lipidmengen die Bildung von Lipidgelen (0/W-Cremes) beschrieben [Freitas, C., Müller, R.H., Effect of light and temperature on zeta potential and physical stability in Solid Lipid Nanoparticle (SLN™) Dispersions, Int. J. Pharm., 221 - 229, 1998]. Bei der Herstellung von Lipidnanopartikeln durch Präzipitation [Gasco, M.R., Method for producing solid lipid microspheres having a narrow size distribution, UA-A-5,250,236, 1993] wird eine heiße Lipidmikroemulison in eine kalte wäßrige Tensidlösung gegeben. Dieser Ausfällungsschritt bedingt zwangsläufig stark verdünnte Lipidnanopartikeldispersionen. Die maximal erreichbare Konzentration in der wäßrigen Dispersion ist laut Patent 0,5 - 3 % [Gasco, M.R., Method for producing solid lipid microspheres having a narrow size distribution, US-A-5,250,236, 1993].

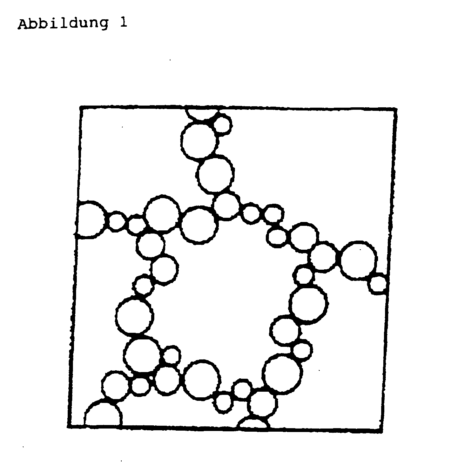

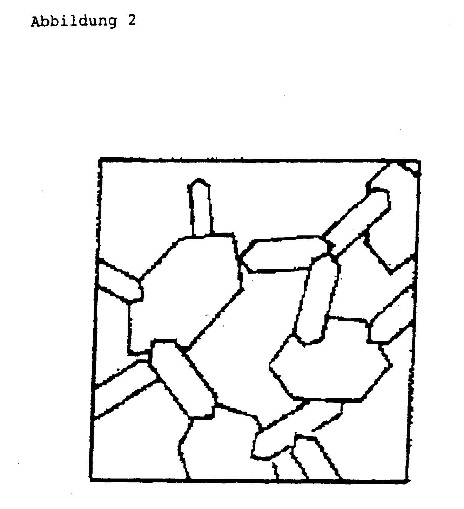

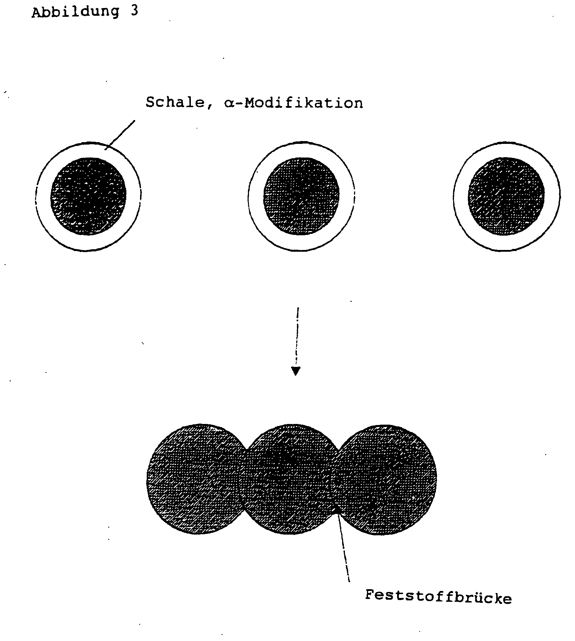

Ziel der bisherigen Verfahren war es, in der Größe homogene Partikeldispersionen zu erzeugen. Homogene Partikeldispersionen werden gerade in den Patenten hervorgehoben, die Partikelherstellung über Homogenisationsverfahren durchführen. Homogene Partikel neigen bei Dispergierung in einem Homogenisationsmedium jedoch dazu, sich "perlschnurartig" aufzureihen und Gele zu bilden. Klassisches Beispiel ist die Gelbildung von größenmäßig einheitlichen Aerosilpartikeln, die sowohl in wäßrigen als auch nicht-wäßrigen Medien (z.B. Öle) auftritt (Abb. 1). Diese perlschnurartigen Gele sind in den Lehrbüchern beschrieben [List, P.H., Arzneiformenlehre, Wissenschaftliche Verlagsgesellschaft Stuttgart, S. 264, 1976]. Eine analoge Gelbildung wird jedoch auch für Partikel beschrieben, die herstellungsbedingt relativ polydispers sind. Klassisches Beispiel hierfür sind die in den Lehrbüchern beschriebenen Bentonit-Gele [List, P.H., Arzneiformenlehre, Wissenschaftliche Verlagsgesellschaft Stuttgart, S. 264, 1976] (Abb. 2). Die Bindungen innerhalb des Gelgerüstes von Bentonit und Aerosil sind nicht kovalent, sondern rein elektrostatisch und/oder Wasserstoffbrückenbindungen. Obwohl keine kovalenten Bindungen vorliegen, sind die Gelgerüste relativ stabil, bereits geringe Konzentrationen ergeben ein hochviskoses Gel (z.B. 2 % Aerosil in Miglyol 812). Bei Lipidnanopartikeln wurde gefunden, daß sie eine dünne äußere Hülle mit unterschiedlicher Struktur zum Partikelkern besitzen [zur Mühlen, A., Schwarz, C., Mehnert, W., Solid Lipid Nanoparticles (SLN) for controlled drug delivery - Drug release and release mechanism, Eur. J. Pharm. Biopharm. 45, 149, 1998, Lukowski, G. , Werner, U., Pflegel, P., surface investigation and drug release of drugloaded solid lipid nanoparticles, Proc. 2nd World Meeting APGI/APV, Paris, 573 - 574, 1998]. Kommen Partikel miteinander in Kontakt, so wandelt sich flüssig-kristalline $\alpha$-Modifikation in feste ß-Modifikation um, es bilden sich Lipidfeststoffbrücken und es entstehen Partikelaggregate (Abb. 3). Mit fortschreitender Partikelaggregation bildet sich ein hochfestes Gel [Freitas, C., Müller, R.H., Correlation between long-term stability of solid lipid nanoparticles (SLN™) and crystallinity of the lipid phase, Eur. J. Pharm. Biopharm., 47, 125 - 132, 1999]. Es wurde gefunden, daß dieser Gelierungs- und Gelbildungsprozeß um so stärker ist, je höher die Konzentration an Lipidpartikeln ist. Die untersuchten Lipidpartikelkonzentrationen waren relativ gering von 0,1 % - 10 % [Freitas, C., Müller, R.H., Correlation between long-term stability of solid lipid nanoparticles (SLN™) and crystallinity of the lipid phase, Eur. J. Pharm. Biopharm., 47, 125 - 132, 1999, Freitas, C., Lucks, J.S., Müller, R.H., Effect of storage conditions on long-term stability of "Solid Lipid Nanoparticles" (SLN) in aqueous dispersion, Proc. 1st World Meeting APGI/APV, Budapest, 493 - 494, 1995]. Lipidpartikel werden quasi bei Gelbildung zusätzlich über Lipidfeststoffbrücken "verklebt".

Angesichts der oben beschriebenen Gelbildungsphänomene von monodispersen und polydispersen Partikeln, des Mechanismus der Feststoffbrückenbildung bei hochfeinen Lipidpartikeln und der raschen Umwandlungsgeschwindigkeit von $\alpha$-Modifikation in ß-Modifikation, d.h. innerhalb von Minuten z.B. bei Hartfett [Sucker, H., Fuchs, P., Speiser, P., Pharmazeutische Technologie, Georg Thieme Verlag Stuttgart, 1978, Bauer, K.H., Frömming, K.-H., Führer, C., Pharmazeutische Technologie, G. Fischer Verlag Stuttgart, S. 276, 1997], erschien die Herstellung hochkonzentrierter Lipiddispersionen in einer Größe von wenigen Mikrometern und insbesondere Nanometern nicht realisierbar. Überraschender Weise wurde jedoch gefunden, daß eine 40%ige hochdruckhomogenisierte Lipiddispersion separate Nanopartikel enthielt (Beispiel 1). Auch bei weiterer Erhöhung des Lipidgehaltes auf 50 % konnten noch separate Nanopartikel erhalten werden (Beispiel 2). Zur Herstellung von Lipidpartikeln im oberen Nanometerbereich bzw. unteren Mikrometerbereich wurde als Dispergiersystem mit niedrigerer Leistungsdichte ein Rotor-Stator (Ultra-Turrax, Janke & Kunkel, Deutschland) eingesetzt (Beispiel 3). Die Herstellung von Lipidpartikeldispersionen einheitlicher Partikelgröße (monodispers) mit einem Feststoffgehalt deutlich oberhalb 74 % ist physikalisch nicht möglich. Bei dichtester Kugelpackung beträgt das Feststoffvolumen 74 %, die Poren dazwischen (im Fall der Lipidpartikeldispersion die Wasserphase) 24 %. Um eine dichtere Packung zu ermöglichen, wurden daher gezielt Lipidpartikeldispersionen uneinheitlicher Partikelgröße hergestellt. Hierzu wurden gezielt entgegen üblicher Lehrmeinung suboptimale Dispergierbedingungen (niedriger Druck) oder suboptimale Dispergiergeräte (uneinheitliche Leistungsdichtenverteilung) eingesetzt (Beispiel 4). Es wurde genau das Gegenteil von den Herstellbedingungen und Herstellgeräten eingesetzt, die in der Literatur zur Herstellung von Lipidpartikeldispersionen empfohlen werden. Uneinheitliche Größe erlaubt eine dichtere Packung bei gleichbleibendem Abstand der Partikel zueinander, da kleinere Partikel sich in die Lücken zwischen größeren Partikeln einpassen können, wobei überraschenderweise die Partikelintegrität erhalten blieb.

Die Feststoffkonzentration der in dieser Erfindung beschriebenen Lipidpartikeldispersionen liegt vorzugsweise im Bereich von 30 % bis zu 95 %. Mit zunehmendem Feststoffgehalt müssen die Herstellbedingungen suboptimaler werden, d.h. die erzeugte Partikeldispersion polydisperser. Im oberen Konzentrationsbereich muß zusätzlich die Lipidphase in mehreren Schritten sukzessive dazugegeben werden. Der schrittweise zugegebene Lipidanteil wird in Anwesenheit von bereits vorhandenen Lipidnanopartikeln in der Wasserphase fein dispergiert. Nach erfolgreicher Dispergierung und Überführung in Lipidnanopartikel wird ein weiterer Lipidanteil zugefügt. So können bei Herstellung von 100 g einer 80% Lipidpartikeldispersion anstelle der Zugabe von direkt 80 g Lipid zu 20 g Wasser zunächst 20 g Lipid zu 20 g Wasser gegeben (= 50%ige Dispersion) und anschließend in 6 weiteren Teilschritten jeweils weitere 10 g Lipid zugegeben werden. Bei jedem Schritt erfolgt somit die Dispergierung der 10 g Lipid in 20 g Wasser und automatisch aufgrund der Volumenverhältnisse die Bildung eines O/W-Systems.

Die Dispergierung des Lipids in der äußeren Phase kann entweder im festen Zustand (Kalthomogenisation) oder im flüssigen Zustand (Heißhomogenisation) erfolgen. Bei der Kalthomogenisation wird das Lipid in einer wäßrigen Tensidlösung dispergiert (Rohdispersion) und dann mit einem entsprechenden Gerät weiterverarbeitet. Bei der Heißhomogenisation wird das Lipid geschmolzen und in die auf gleicher Temperatur erhitzte äußere Phase gegossen und darin dispergiert (Rohemulsion). Die erhaltene Rohemulsion wird dann mit einem weiteren Dispergiergerät bearbeitet. In Abhängigkeit vom gewünschten Dispergiergrad, der Konzentration der Lipidphase und dem Aggregatzustand des Lipids werden als Dispergiersysteme eingesetzt: Hochdruckhomogenisatoren vom Typ des Kolben-Spalt-Homogenisators (APV Gaulin Systeme, French Press).

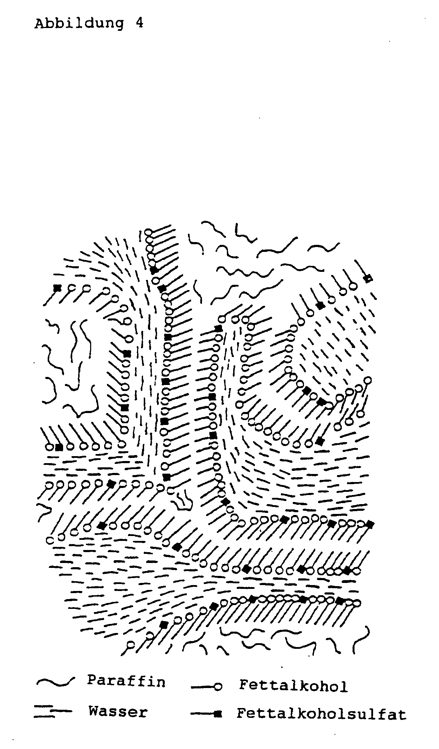

Insbesondere bei der Dispergierung von hochkonzentrierten geschmolzenen Lipiden (Heißhomogenisation) wurde davon ausgegangen, daß sich die typischen in den Lehrbüchern beschriebenen biamphiphilen Creme-Strukturen bilden [Bauer, K.H., Frömming, K.-H., Führer, C., Pharmazeutische Technologie, G. Fischer Verlag Stuttgart, S. 276, 1997] (Abb. 4). Aus derartigen Strukturen können keine Partikel wie z.B. Nanopartikel mehr erhalten werden. In der vorliegenden Erfindung wurden jedoch überraschenderweise auch bei hoher Lipidkonzentration integere Partikel erhalten.

Die Partikelbildung unter Minimierung von Partikelaggregaten kann durch Zusätze gefördert werden. Derartige Zusätze sind Substanzen, die den pH-Wert verschieben (z.B. Erhöhung des Zetapotentials, Beeinflussung der Tensidstruktur wie Dissoziationsgrad) oder gezielt die Partikelladung erhöhen (z.B. Anti-Flokkulantien wie Natriumcitrat). Derartige Zusätze können auch die Stabilität der Lipidpartikeldispersion erhöhen, z.B. über Beeinflussung von Wasserstruktur (z.B. Elektrolyte) oder Effekte auf die stabilisierende Tensidschicht (z.B. Glukose bei Lecithin).

Die Lipidpartikel können mit Wirkstoffen beladen werden. Wirkstoffe sind z.B. Arzneistoffe, kosmetische Wirkstoffe, Pflanzenschutzmittel, Lebensmittelzusatzstoffe, chemische Substanzen unterschiedlicher Art (z.B. Holzschutzmittel).

Die Beladung mit Wirkstoffen kann auf verschiedenen Wegen, einzeln oder in Kombination erfolgen. Der oder die Wirkstoffe sind in den Lipidteilchen gelöst, lösungsvermittelt (z.B. mit Tensiden oder Cyclodextrinen) oder dispergiert. Ferner können sie an deren Oberfläche adsorbiert sein. Aufgrund des Feststoffcharakters können auch hydrophile Wirkstoffe in Form einer wäßrigen Wirkstofflösung in die Lipid- oder Lipidphase eingearbeitet werden. Nach dieser Einarbeitung und der anschließenden Dispergierung des Lipids in dem wäßrigen Dispersionsmedium entsteht ein System W/F/W, d.h. Wasser in Fett in Wasser. Der Lipoidkern schließt hierbei die wäßrige Arzneistofflösung aufgrund seines festen Aggregatzustandes besser ein als es bei vergleichbaren multiplen Emulsionen Wasser in Öl in Wasser (W/Ö/W) möglich ist.

Die erfindungsgemäßen Lipidpartikel können auf folgende Weisen hergestellt werden:

1. Dispergieren der inneren Phase (des Lipids oder Lipoids) in geschmolzenem oder erweichtem Zustand. Die Dispergierung erfolgt oberhalb der Raumtemperatur und kann durch verschiedene, beispielsweise die unten beschriebenen Verfahren bewirkt werden.

2. Dispergieren der festen inneren Phase in festem Zustand. Die feste Phase wird hierfür fein zerkleinert und in Wasser oder in einem wäßrigen Medium dispergiert.

Der dispergierte, bei Raumtemperatur feste Lipidkern wurde zuvor mit einem oder mehreren Wirkstoffen beladen. Dies kann dadurch erfolgen, daß der Wirkstoff in dem Lipid gelöst oder dispergiert wird, an dessen Oberfläche adsorbiert wird oder in Form einer wäßrigen Lösung in dem Lipid dispergiert wird oder gleichzeitig nach mehreren dieser Methoden eingearbeitet wird.

Die Einarbeitung des oder der Wirkstoffe kann nach verschiedenen Methoden erfolgen. Beispielhaft seien genannt:

1. Lösen des Wirkstoffs in der inneren Phase.
2. Lösen des Wirkstoffs in einem mit der inneren Phase mischbaren Lösungsmittel und Zugabe dieser Wirkstofflösung zur inneren Phase. Anschließend wird gegebenenfalls das Lösungsmittel teilweise oder vollständig entfernt.
3. Dispergieren des Wirkstoffs in der inneren Phase (z.B. durch Dispergieren eines Feststoffs oder gezielte Präzipitation).
4. Lösen des Wirkstoffs in der äußeren, wäßrigen Phase (z.B. amphiphile Substanzen) und Einbindung des Wirkstoffs in einen die Teilchen stabilisierenden Tensidfilm während der Herstellung.
5. Adsorption des Wirkstoffs an der Teilchenoberfläche.
6. Lösen des Wirkstoffs in der Lipidphase mittels eines Lösungsvermittlers (z.B. eines Blockcopolymeren oder Sorbitanfettsäureesters), anschließende Dispergierung der Lipidphase zur Herstellung der Vordispersion. Der Wirkstoff liegt dann in den Partikeln als feste Lösung vor.
7. Einarbeiten von wäßrigen Wirkstofflösungen in die Lipidphase und anschließende Dispergierung der Lipidphase zur Herstellung der Vordispersion, so daß ein System W/F/W entsteht, das den multiplen Emulsionen analog ist.

Es können Wirkstoffe z.B. aus folgenden chemischen Verbindungsklassen eingearbeitet werden:

- hydroxylierte Kohlenwasserstoffe
- Carbonylverbindungen wie Ketone (z.B. Haloperidol), Monosaccharide, Disaccharide und Aminozucker
- Carbonsäuren wie aliphatische Carbonsäuren, Ester aliphatischer und aromatischer Carbonsäuren, basisch substituierte Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide (z.B. Ciclosporin), Polypeptide, ß-Lactamderivate, Penicilline, Cephalosporine, aromatische Carbonsäuren (z.B. Acetylsalicylsäure), Amide aromatischer Carbonsäuren, vinyloge Carbonsäuren und vinyloge Carbonsäureester
- Kohlensäurederivate wie Urethane und Thiourethane, Harnstoff und Harnstoffderivate, Guanidinderivate, Hydantoine, Barbitursäurederivate und Thiobarbitursäurederivate

- Nitroverbindungen wie aromatische Nitroverbindungen und heteroaromatische Nitroverbindungen
- Amine wie aliphatische Amine, Aminoglykoside, Phenylalkylamine, Ephedrinderivate, Hydroxyphenylethanolamine, Adrenalinderivate, Amfetaminderivate, aromatische Amine und Derivate, quartäre Ammmoniumverbindungen
- schwefelhaltige Verbindungen wie Thiole und Disulfane
- Sulfone, Sulfonsäureester und Sulfonsäureamide
- Polycarbocyclen wie Tetracycline, Steroide mit aromatischem Ring A, Steroide mit alpha, beta-ungesättigter Carbonylfunktion im Ring A und alpha Ketol-Gruppe (oder Methylketo-Gruppe) am C 17, Steroide mit einem Butenolid-Ring am C 17, Steroide mit einem Pentadienolid-Ring am C 17 und Seco-Steroide
- 0-haltige Heterocyclen wie Chromanderivate (z.B. Cromoglicinsäure)
- N-haltige Heterocyclen wie Pyrazolderivate (z.B. Propyphenazon, Phenylbutazon)
- Imidazolderivate (z.B. Histamin, Pilocarpin), Pyridinderivate (z.B. Pyridoxin, Nicotinsäure), Pyrimidinderivate (z.B. Trimetoprim), Indolderivate (z.B. Indometacin), Lysergsäurederivate (z.B. Ergotamin), Yohimbanderivate, Pyrrolidinderivate, Purinderivate (z.B. Allopurinol), Xanthinderivate, 8-Hydroxychinolinderivate, Aminohydroxyalkylierte Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin) und Dibenzazepinderivate (z.B. Trimipramin)
- S-haltige Heterocyclen wie Thioxanthenderivate (z.B Chlorprothixen)
- N,0- und N,S-haltige Heterocyclen wie monocyclische N,0-haltige Heterocyclen, monocyclische N,S-haltige Heterocyclen, Thiadiazinderivate, bicyclische N,S-haltige Heterocyclen, Benzothiadiazinderivate, tricyclische N,S-haltige Heterocyclen und Phenothiazinderivate
- 0,P,N-haltige Heterocyclen (z.B. Cyclophosphamid).

An Arzneistoffen können insbesondere folgende Gruppen und Substanzen, z.B. als Salz, Ester, Ether oder in der freien Form, eingearbeitet werden:

Analgetika/Antirheumatika
BTM Basen wie Morphin, Codein, Piritamid, Fentanyl und Fentanylderivate, Levomethadon, Tramadol, Diclofenac, Ibuprofen, Indometacin, Naproxen, Piroxicam, Penicillamin

Antiallergika
Pheniramin, Dimetinden, Terfenadin, Astemizol, Loratidin, Doxylamin, Meclozin, Bamipin, Clemastin

Antibiotika/Chemotherapeutika
hiervon: Polypeptidantibiotika wie Colistin, Polymyxin B, Teicplanin, Vancomycin; Malariamittel wie Chinin, Halofantrin, Mefloquin, Chloroquin, Virustatika wie Ganciclovir, Foscarnet, Zidovudin, Aciclovir und andere wie Dapson, Fosfomycin, Fusafungin, Trimetoprim

Antiepileptika
Phenytoin, Mesuximid, Ethosuximid, Primidon, Phenobarbital, Valproinsäure, Carbamazepin, Clonazepam

Antimykotika

   a) intern:

      Nystatin, Natamycin, Amphotericin B, Flucytosin, Miconazol, Fluconazol, Itraconazol

   b) extern außerdem:

      Clotrimazol, Econazol, Tioconazol, Fenticonazol, Bifonazol, Oxiconazol, Ketoconazol, Isoconazol, Tolnaftat

Corticoide (Interna)
Aldosteron, Fludrocortison, Betametason, Dexametason, Triamcinolon, Fluocortolon, Hydroxycortison, Prednisolon, Prednyliden, Cloprednol, Methylpredinsolon

Dermatika

   a) Antibiotika:

Tetracyclin, Erythromycin, Neomycin, Gentamycin, Clindamycin, Framycetin, Tyrothricin, Chlortetracyclin, Mipirocin, Fusidinsäure

b) Virustatika wie oben, außerdem:

Podophyllotoxin, Vidarabin, Tromantadin

c) Corticoide wie oben, außerdem:

Amcinonid, Flupredniden, Alclometason, Clobetasol, Diflorason, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid

Diagnostika

a) radioaktive Isotope wie Te99m, In111 oder I131, kovalent gebunden an Lipide oder Lipoide oder andere Moleküle oder in Komplexen
b) hochsubstituiertre iodhaltige Verbindungen wie z.B. Lipide

Hämostyptika/Antihämorrhagika
Blutgerinnungsfaktoren VIII, IX

Hypnotika, Sedativa
Cyclobarbital, Pentobarbital, Phenobarbital, Methaqualon (BTM), Benzodiazepine (Flurazepam, Midazolam, Nitrazepam, Lormetazepam, Flunitrazepam, Triazolam, Brotizolam, Temazepam, Loprazolam)

Hypophysen-, Hypothalamushormone, regulatorische Peptide und ihre Hemmstoffe
Corticotrophin, Tetracosactid, Choriongonadotropin, Urofollitropin, Urogonadotropin, Somatropin, Metergolin, Bromocriptin, Terlipressin, Desmopressin, Oxytocin, Argipressin, Ornipressin, Leuprorelin, Triptorelin, Gonadorelin, Buserelin, Nafarelin, Goselerin, Somatostatin

Immuntherapeutika und Zytokine
Dimepranol-4-acetatamidobenzoat, Thymopentin, $\alpha$-Interferon, ß-Interferon, $\gamma$-Interferon, Filgrastim, Interleukine, Azathioprin, Ciclosporine

Lokalanaesthetika
intern:

Butanilicain, Mepivacain, Bupivacain, Etidocain, Lidocain, Articain, Prilocain,

extern außerdem:

Propipocain, Oxybuprocain, Tetracain, Benzocain

Migränemittel
Proxibarbal, Lisurid, Methysergid, Dihydroergotamin, Clonidin, Ergotamin, Pizotifen

Narkosemittel
Methohexital, Propofol, Etomidat, Ketamin, Alfentanil, Thiopental, Droperidol, Fentanyl

Nebenschilddrüsenhormone, Calciumstoffwechselregulatoren
Dihydrotachysterol, Calcitonin, Clodronsäure, Etidronsäure

Opthalmika
Atropin, Cyclodrin, Cyclopentolat, Homatropin, Tropicamid, Scopolamin, Pholedrin, Edoxudin, Idouridin, Tromantadin, Aciclovir, Acetazolamid, Diclofenamid, Carteolol, Timolol, Metipranolol, Betaxolol, Pindolol, Befunolol, Bupranolol, Levobununol, Carbachol, Pilocarpin, Clonidin, Neostimgin

Psychopharmaka
Benzodiazepine (Lorazepam, Diazepam), Clomethiazol

Schilddrüsentherapeutika
1-Thyroxin, Carbimazol, Thiamazol, Propylthiouracil

Sera, Immunglobuline, Impfstoffe

a) Immunglobuline allgemein und spezifisch wie Hepatitis-Typen, Röteln, Cytomegalie, Tollwut, FSME, Varicella-Zoster, Tetanus, Rhesusfaktoren

b) Immunsera wie Botulismus-Antitoxin, Diphterie, Gasbrand, Schlangengift, Skorpiongift

c) Impfstoffe wie Influenza, Tuberkulose, Cholera, Diphterie, Hepatitis-Typen, FSME, Röteln, Hämophilus influenzae, Masern, Neisseria, Mumps, Poliomyelitis, Tetanus, Tollwut, Typhus

Sexualhormone und ihre Hemmstoffe
Anabolika, Androgene, Antiandrogene, Gestagene, Estrogene, Antiestrogene (Tamoxifen etc.)

Zystostatika und Metastasenhemmer

a) Alkylantien wie Nimustin, Melphalan, Carmustin, Lomustin, Cyclophosphamid, Ifosfamid, Trofosfamid, Chlorambucil, Busulfan, Treosulfan, Prednimustin, Thiotepa

b) Antimetabolite wie Cytarabin, Fluorouracil, Methotrexat, Mercaptopurin, Tioguanin

c) Alkaloide wie Vinblastin, Vincristin, Vindesin

d) Antibiotika wie Aclarubicin, Bleomycin, Dactinomycin, Daunorubicin, Doxorubicin, Epirubicin, Idarubicin, Mitomycin, Plicamycin

e) Komplexe von Nebengruppenelementen (z.B. Ti, Zr, V, Nb, Ta, Mo, W, Ru, Pt) wie Carboplatin, Cisplatin und Metallocenverbindungen wie Titanocendichlorid

f) Amsacrin, Dacarbazin, Estramustin, Etoposid, Hydroxycarbamid, Mitoxanthron, Procarbazin, Temiposid

g) Alkylamidophospholipide (beschrieben in J.M. Zeidler, F. Emling, W. Zimmermann und H.J. Roth, Archiv der Pharmazie, 324 (1991), 687)

h) Etherlipide wie Hexadecylphosphocholin, Ilmofosin und Analoga, beschrieben in R. Zeisig, D. Arndt und H. Brachwitz, Pharmazie 45 (1990), 809-818.

i) Taxane wie z.B. Paclitaxel.

Die konzentrierten Lipidpartikeldispersionen eignen sich wie oben ausgeführt besonders wegen ihres geringen Wassergehaltes zur Herstellung diverser Arzneiformen wie z.B. Granulate (z.B. Abfüllung in Sachets), Tabletten, Pellets, Kapseln, Trockenprodukte wie Lyophilisate und sprühgetrocknete Produkte. Essentieller Vorteil ist, daß nur geringe Wassermengen entfernt werden müssen. Dies reduziert Prozeßzeit, Kosten und vor allem kommt es aufgrund geringerer Prozeßzeit auch zu weniger Aggregation von Partikeln.

Weiterhin können die konzentrierten Lipidpartikeldispersionen aufgrund bereits ausreichend hoher Viskosität direkt als topische Arzneiform eingesetzt werden (z.B. Gel) oder nach Zusatz von viskositätserhöhenden Substanzen oder flüssiger öliger Phase.

Die Dispersionen können, gegebenenfalls nach Verdünnen mit Wasser, mit handelsüblichen Geräten versprüht werden (z.B. nasale Applikation) oder als Aerosol vernebelt werden, z.B. mit einem Pariboy (Beispiel 9) oder HaloLite™ (Firma Medic-Aid, England). Weitere Einsatzmöglichkeit ist die Herstellung parenteraler Arzneiformen, insbesondere wenn das zu applizierende Volumen minimal gehalten werden soll. Kleine Volumina können aufgrund der hohen Konzentration an Lipidpartikeln realisiert werden. Die Lipidpartikel können aseptisch produziert oder nachträglich mit üblichen Verfahren sterilisiert werden.

Weitere Wirkstoffe zur Einarbeitung in die erfindungsgemäßen Lipidpartikel sind Duftstoffe jeglicher Art, natürlichen, synthetischen oder halbsynthetischen Ursprungs. Als Beispiele dienen ätherische Öle wie Zitronenöl (Beispiel X1), Rosenöl, Lavendelöl, Bergamottöl, Melissenöl, Nelkenöl, Zimtöl, Orangenöl, Jasminöl, Rosmarinöl, Anisöl, Pfefferminzöl, Sandelholzöl oder Ylang-Ylang-Öl, deren isolierte Inhaltsstoffe wie z.B. 1,8-Cineol, Menthol, Terpinhydrat, Limonen, α-Pinen, Eugenol sowie Parfüms, insbesondere Parfümöle. Eingesetzt werden können alle auf dem Markt befindlichen Parfüms, z. B. Allure (Beispiel X2), Coco, Egoiste, Chanel No. 5, 19, 22 von Chanel, Miss Dior, Dune, Diorissime oder Fahrenheit von Dior, Roma, Laura, Venezia von Laura Biagotti, L' air du temps von Nina Ricci, Chalimar von Guerlain, Tresor von Lancome, Gio von Armani, Escape, Obsession, CK One, CK be, Eternity von Calvin Klein, Berlin, Joop, Rococo, All about Eve, What about Adam, Nightflight von Joop, KL, Lagerfeld, Jako von Karl Lagerfeld, Extreme von Bulgari.

Neben Duftstoffen mit angenehmem Duft können auch Duftstoffe mit abstoßender Wirkung eingearbeitet werden, z.B. Repellents. Duftstoffe mit abstoßender Wirkung können z.B. als Warnstoffe (Schutz vor versehentlicher oraler Einnahme des Produktes) oder als Repellents, z.B. gegen Insekten eingearbeitet werden. Beispiele für natürliche Repellents sind Citrusöle, Eukalyptusöl und Campher, Beispiele für synthetische Repellents sind N,N-Diethyl-toluamid (DEET), Dibutylphthalat, Dimethylphthalat, 2-Ethyl-1,3-hexandiol.

Weitere Duftstoffe sind die sog. Lockstoffe wie z.B. Pheromone. Lipidpartikel mit Lockstoffen können z.B. Einsatz in Kosmetika oder in Insektenvernichtungsmittel finden. Beispiele für Pheromone sind Androstenon und Androstenol; insbesondere werden menschliche Pheromone verwendet.

Zur Insektenvernichtung oder Abtötung von Kleinlebewesen (z.B. Flöhe, Läuse) können die Lipidpartikel neben Lockstoffen gleichzeitig auch als Wirkstoff Gifte enthalten. Alternativ können Lockstoff-haltige Lipidpartikel mit Lipidpartikeln gemischt werden, die Gift enthalten. Neben Giften, die eine orale Aufnahme durch das Insekt/Kleinlebewesen verlangen, kommen auch Kontaktgifte zum Einsatz. Aufgrund ihres lipophilen Charakters kommt es z.B. zur Adhäsion der Lipidpartikel am lipophilen Chitinpanzer von Insekten, das Kontaktgift diffundiert aus den anhaftenden Partikeln in das Insekt. Beispiele für Gifte sind chlorierte Kohlenwasserstoffe wie γ-Hexachlorcyclohexan, Pyrethrine, Pyrethroide, Alkylphosphate wie Paraoxon, Parathion, Fenthion, Dichlorvos und Carbaminsäureester wie Butoxicarboxim, Bendiocarb, Methomyl, Proxopur.

Bei der Einarbeitung öliger Duftstoffe stellen diese Öle die flüssige Lipidkomponente in den Lipidpartikeln dar, das heißt der flüssige Duftstoff wird mit einem bei Raumtemperatur festen Lipid gemischt, zusätzlich können noch ein oder mehrere flüssige Lipide zugemischt werden. Duftstoffe können auch in einem flüssigen Lipid gelöst und anschließend zur Partikelherstellung mit festem Lipid verschnitten werden. Alternativ ist auch Lösung im geschmolzenen festen Lipid möglich.

Durch Einarbeitung der Duftstoffe in die Lipidpartikel kann deren Freisetzung kontrolliert werden, insbesondere kann kontrolliert eine verlängerte Freisetzung erzielt werden (prolonged release). So setzen Produkte auf der Basis von Emulsionen (öliger Duftstoff in Wasser emulgiert, Duftstoff in der Ölphase einer 0/W-Emulsion gelöst) den Duftstoff relativ schnell frei. Das Produkt verliert seine Duftwirkung. Einarbeitung in die Lipidpartikel reduziert die Abgabe an Duftstoffen und verlängert somit die Produktwirksamkeit (Beispiele 18 und 19).

Anwendungsbeispiele für angenehme Duftstoffe sind kosmetische Produkte (Lotionen, After Shaves etc.), pharmazeutische Produkte (Steigerung der Akzeptanz beim Patienten) sowie Produkte für Hygiene- und Sanitärbereich (z.B. Beduftung von Räumen über einen längeren Zeitraum).

Weitere Wirkstoffe zur Einarbeitung in die erfindungsgemäßen Lipidpartikel sind Marker (Label) unterschiedlicher Art. Beispiele für derartige Markierungsstoffe sind radioaktive Substanzen, zum Beispiel jodierte Lipide (jodiert z. B. mit Jod 131, Jod 123) sowie lipophile Indiumverbindungen (z. B. Indium-111Oxin (8-Hydroxy-Chinolin)) sowie Technetium 99m, welche molekular eingebunden oder komplexgebunden oder adsorbiert sind. Derartige Partikel können zur Gammaszintigraphie eingesetzt werden, z. B. nach intravenöser Injektion zur Markierung von Knochenmark und Leber. Andere Marker sind farbige Substanzen (Farbstoffe) oder fluoreszierende Substanzen (Fluoreszenzfarbstoffe).

Ein Beispiele für farbige Substanzen ist Sudanrot. Beispiele für fluoreszierende Substanzen sind z. B. Nile red und Fluorescein. Generell können mit Markern versehene Lipidpartikel sowohl zur in-vitro Diagnostik als auch in-vivo Diagnostik eingesetzt werden. Beispiele für in-vitro Diagnostik sind die Charakterisierung von Zellinien, z. B. Bestimmung der Phagozytoseaktivität nach Differenzierung einer Zellinie in eine Makrophagenlinie. Ebenso können diese Partikel in diagnostischen Kits verwendet werden. Beispiele für in-vivo Diagnostik sind die Markierung von Lymphknoten. Hierzu werden die Partikel in der Nähe von Lymphknoten injiziert, es erfolgt Drainage in die Lymphknoten und Anfärbung. Ein weiteres Beispiel ist die Markierung von Körperhöhlen und Analytik mit Fluoreszenzspektroskopie.

Die erfindungsgemäßen Lipidpartikel können auch ohne Einarbeitung von Wirkstoffen als Diagnostika in der Magnetresonanztomographie (MR) eingesetzt werden. Obwohl durch die Einführung der Magnetresonanztomographie die bildgebende abdominale Diagnostik entscheidend verbessert werden konnte, ist die sichere Abgrenzung des Gastrointestinaltraktes von normalem oder pathologischen Umgebungsgewebe auch heute noch oft schwierig oder unmöglich. MR-Aufnahmen sind prinzipiell ohne Kontrastmittel möglich, die Signalintensitäten und damit die Stärke des Kontrastes werden jedoch durch die Relaxationszeit beeinflußende T1- und T2-Kontrastmittel verbessert. Um die Abgrenzung der Gewebestrukturen zu verbessern, wurden unterschiedliche Substanzen als Kontrastmittel vorgeschlagen. Prinzipiell läßt sich dabei eine Einteilung in negative Kontrastmittel (Magnetite) und positive Kontrastmittel (z.B. Magnevist) vornehmen. Alle bisher getesteten Substanzen können den Anforderungskatalog (niedriger Preis, gute Akzeptanz und Verträglichkeit, fehlende Toxizität, keine Induktion von Bewegungsartefakten, Resistenz gegenüber pH, homogene Verteilung im gesamten Gastrointestinaltrakt, gutes Anschmiegen an die Darmwand, Konrastgebung in allen Pulssequenzen) nur bedingt erfüllen. SLN sind durch ihren hohen Fettanteil orale T1-MR Kontrastmittel. Vorteile sind niedriger Preis, gute Akzeptanz (Geschmack, Verträglichkeit), Verträglichkeit, fehlende Toxizität (physiologische Lipide), homogene Verteilung im Gastrointestinaltrakt und gutes Auskleiden der Darmwand (Partikelgröße).

Von den mittels Bruker minispec pc120 getesteten SLN Dispersionen eignen sich z. B. Witepsol H15 (Hartfett C12-C18) und Witepsol E85 (Hartfett C12-C18) wegen ihrer Beeinflussung der T1-Relaxationszeiten als MR Kontrastmittel.

Als Marker können in die erfindungsgemäßen Lipidpartikel auch Magnetite ($Fe_2O_3$, Eisenoxide) eingearbeitet werden. Insbesondere werden kleine Eisenoxidpartikel im Bereich ca. 1 bis 3 nm in die Lipidmatrix eingearbeitet. Sie können ebenfalls für die Magnetresonanztomographie als Kontrastmittel verwendet werden.

Die oben beschriebene Umwandlung von Lipiden der Lipidpartikelmatrix in die hochgeordnete stabile β-Modifikation führt zu einer physikalischen Destabilisierung, d.h. zu Partikelaggregaten (über z.B. Feststoffbrückenbildung). Dies wird bei den erfindungsgemäßen flüssig-fest Partikeln dadurch verhindert, daß ein Teil des Lipids in einem hoch ungeordneten Zustand sich befindet, d.h. flüssiger Zustand/Schmelze (z.B. Miglyol, Beispiel 12) oder in einem de facto flüssigen Zustand (α-Modifikation). Die α-Modifikation besitzt eine geringe Packungsdichte (K. Thoma, P.Serno, D. Precht, Röntgendiffraktometrischer Nachweis der Polymorphie von Hartfett, Pharm. Ind 45, 420-425, 1983), die Fettsäurereste können relativ frei oszillieren, so daß der Zustand ähnlich einer Schmelze ist (L. Hernqvist, Crystal structures of fats and fatty acids, in: N. Garti, K. Sato, Crystallisation and polymorphism of fats and fatty acids, Marcel Dekker Inc., New York, Basel, 97-138, 1988).

Die Erfindung nutzt auch aus, daß bei komplex zusammengesetzten Fetten (z.B. Hartfett) auch unterhalb des Schmelzpunktes ein gewisser Anteil in der flüssigen Form vorliegt (I. Hassan, Phasenverhalten langkettiger Glyceride, Dissertation, Christian-Albrechts-Universität Kiel, 1988). Bisheriges Problem war jedoch, daß dieser flüssige Anteil die Umwandlung der metastabilen in die stabile β-Modifikation fördert (J. Schlichter-Aronhime, N. Garti, Solidification and polymorphism in cacoa butter and the blooming problems, in: N. Garti, K. Sato, Crystallisation and polymorphism of fats and fatty acids, Marcel Dekker Inc., New York, Basel, 363-392, 1988; H. Yoshino, M. Kobayashi, M. Samejima, Influence of fatty acid composition on the properties and polymorphic transition of fatty suppository bases, Chem. Pharm. Bull. 31, 237-246, 1983). In der vorliegenden Erfindung wurde überraschend gefunden, daß

a) dieser Übergang bei entsprechender Zusammensetzung der Partikelformulierung (z.B. Beispiel 13) nicht eintritt oder

b) bei bestimmter Zusammensetzung der Partikelformulierung beschleunigt eintritt (Beispiele 14-16), was erfindungsgemäß zur kontrollierten Freisetzung von Wirkstoffen benutzt werden kann.

Die Ausbildung der stabilen βi/β-Modifikation führt zum unerwünschten Effekt, der Verdrängung von in die Partikelmatrix inkorporierten Wirkstoffen (sog. drug exclusion). In den Lipidpartikeldispersionen bilden sich Wirkstoffkristalle. Beim Übergang vom ungeordneten Zustand (flüssige bzw. flüssigkeitsähnliche α-Modifikation) in die stabilere βi/β-Modifikation verringert sich die Anzahl flüssiger Bereiche mit gelöstem Arzneistoff und die Anzahl der Gitterdefekte (und damit die Möglichkeit zur Unterbringung von Wirkstoffmolekülen in der Lipidmatrix). Es bilden sich perfekte Kristalle, der Wirkstoff wird rausgedrängt. Dies ist besonders ausgeprägt bei reinen monoaciden Triglyceriden, die hochkristalline feste Lipidpartikel bilden (Westesen, K., Bunjes, H., Koch, M.H.J., Physicochemical characterisation of lipid nanoparticles and evaluation of their drug loading capacity and sustained release potential, J. Control. Release 48, 223-236, 1997).

Die erfindungsgemäßen fest-flüssig Lipidpartikel bleiben auch in hochkonzentrierten Dispersionen als individuelle Partikel erhalten, sie weisen nach Herstellung (z.B. Beispiel 6) aber auch nach Lagerung einen flüssigen bzw. α-Anteil auf (Beispiel 13). Der flüssige Anteil kann durch Zumischung von flüssigen Lipiden (Öle, z.B. Triglyceride wie Miglyole) zu festen Lipiden gezielt erhöht werden. Geringe Mengen an Öl können im festen Lipid gelöst werden (d.h. molekulardispers verteilt). Bei Überschreitung der Öllöslichkeit im festen Lipid kommt es zu Ansammlungen ungelöster Ölmoleküle, es bilden sich Kompartimente mit flüssigem Öl im Nanometerbereich (sog. Nano-Compartimente). Neben Fehlstellen im Lipidgitter von weniger perfekter β'-Modifikation kann sich hier in den flüssigen Nano-Compartimenten Wirkstoff in gelöster Form einlagern. Es entsteht somit ein Carrier, der aus einer festen Lipidmatrix mit inkorporierten Nano-Compartimenten aus flüssigem Lipid besteht, der Nano-Compartiment-Carrier (NCC). Konservierung von möglichst viel Lipid in ungeordneter Form in diesem Nano-Compartiment-Carrier und Hemmung der Bildung von βi/β-Modifikation (d.h. Vermeidung der Bildung eines festen Lipidnanopartikels) fördert die Wirkstoffinkorporation.

Durch Mischung von flüssigen und festen Lipiden erhalten die erfindungsgemäßen Partikel eine besondere innere Struktur mit erhöhter Unordnung (flüssige Kompartimente, flüssig-kristalline Anteile, amorphe Strukturen) und sind nicht mehr vollständig kristallin.

Die Kristallinität der Partikel wird ermittelt durch Vergleich ihrer Schmelzenthalpie mit der Schmelzenthalpie der Bulkware des eingesetzten festen Lipids in seiner kristallinen lagerstabilsten Modifikation, der β-Form (= 100 % Kristallinität). Die Bestimmung der Schmelzenthalpie der erfindungsgemäßen Partikel erfolgt direkt nach der Herstellung mit Differential Scanning Calorimetry (DSC) im Vergleich zur Bulkware. Berechnung der Schmelzenthalpie der Partikel im Prozent der Schmelzenthalpie der Bulkware ergibt die Kristallinität in Prozent bzw. den Kristallinitätsindex (z.B. 80 % Kristallinität entspricht dem Kristallinitätsindex 0,80, s.u.).

Vollständig kristallin sind Partikel mit einem Kristalinitätsindex von 1,0, überwiegend kristallin mit einen Kristallinitätsindex von 0,50 oder höher. Überwiegend nicht-kristalline Partikel haben einen Kristallinitätsindex kleiner als 0,50, bei nahezu vollständig röntgenamorphen Lipidpartikeln geht der Kristallinitätsindex gegen Null. Überwiegend kristalline Partikel und überwiegend nicht-kristalline Partikel sind alle teilkristallin, da zumidnest ein Teil der Matrix kristallin vorliegt.

Die Hemmung der Ausbildung der stabilen βi/β-Modifikation kann dadurch erzielt werden, daß - wie oben ausgeführt - ungeordnetes Lipid (= ohne feste, hochgeordnete Kristallstruktur) bei teilkristallinen Partikeln aufgrund eines flüssigen Anteils vorhanden ist. Flüssige Lipide werden mit festen Lipiden gemischt, wobei in den meisten Fällender flüssige Anteil weniger als 50% beträgt. Alternativ kann die erforderliche fehlende Kristallinität auch durch gezielte Herstellung von amorphen Partikeln realisiert werden. Amorphe Strukturen zeigen keine Kristallinität. Die Partikel bilden sich aus, wenn wie oben beschrieben ein festes und flüssiges Lipid gemischt werden, wobei der Anteil des flüssigen Lipids zur Ausbildung dieser Partikel in der Regel mindestens 50 % beträgt und bis 99% ansteigen kann. Das Öl wird quasi verfestigt unter Vermeidung der Ausbildung einer geordneten kristallinen Struktur. Die gebildeten Partikel sind somit ebenfalls teilkristallin (überwiegend nicht-kristallin), bei Temperatur von 21° C halbfest bis fest und röntgenamorph.

Diese Partikel stehen somit im Widerspruch zu in der Literatur beschriebenen Lipidpartikeln hergestellt

a) alleinig aus festen Lipiden oder
b) Partikeln, die überwiegend kristallin sind.

Durch Eldem et al. (Eldem, T:, et al., Optimization of spray dried and congealed lipid micropellets and characterisation of their surface morphology by scanning electron microscopy, Pharm. Res. 8, 47 - 51, 1991) sind Mikropartikel aus festen Lipiden bekannt, die durch Sprühtrocknung oder -erstarrung hergestellt werden. Das Matrixmaterial dieser Partikel besteht ausschließlich aus festen Lipiden. US-A-5 188 837 (Domb A., Lipospere for controlled delivery of substances, 1993) beschreibt Lipid-Mikrosphären, die aus festen Lipid (z.B. einem Wachs) bestehen und mit einer Phospholipidschicht bedeckt sind. Tsutsumi et al. (J. Soc. Cosmet. Chem. 30, 345 - 356, 1979) beschreiben kristalline Mikropartikel aus Hartparaffin, die aber physikalisch instabil sind. Erst als Nanopartikel kann eine ausreichende physikalische Stabilität dieser Hartparaffin Partikel erzielt werden (de Vringer, T., Topical preparations containing a suspension of solid lipid particles, European patent application 0 506 197 Al, 1992). Diese und andere Nano- oder Mikropartikeln aus Lipiden sind stets überwiegend kristallin (z. B. in der α oder β' Kristallmodifikation) . Aufgrund der Kristallinität dieser Partikel ist das Aufnahmevermögen für Wirkstoffe zumeist begrenzt (Westesen, K., et al., Physicochemical characterisation of lipid nanoparticles and evaluation of their drug loading capacity and sustained release potential. J. Control. Rel. 48, 223 - 236, 1997).

Die Mischung von flüssigen und festen Lipiden führt zu ungeordneten Strukturen mit verbesserter Wirkstoffinkorporation, wobei

a) bei einem flüssigen Lipidanteil (Öl) unterhalb von 50% sich vorwiegend ungeordnete flüssige Bereiche (Nano-Compartimente) sich innerhalb eines festen Partikels ausbilden,
b) bei einem flüssigen Lipidanteil von 50% oder mehr das flüssige ungeordnete Lipid durch das feste Lipid verfestigt wird (verfestigtes Öl).

Kennzeichnend für die Erfindung ist, daß ungeordnete und lagerstabile Strukturen durch Zumischung flüssiger Lipide erzeugt werden, die zu teilkristallinen oder überwiegend nicht-kristallinen Partikeln führen, mit halbfestem oder festem Aggregatzustand.

Verfestigte Öle sind aus der DE 197 07 309 A1 bekannt (Clermont-Gallerande, H., Feste kosmetische Zubereitung auf Basis verfestigeter Öle, 1998). Allerdings sind diese Zubereitungen wasserfrei und stellen keine Dispersion dar. Vielmehr wird dieses Produkt in Stiftform angewandt.

Eine Teilaufgabe der vorliegenden Erfindung war es daher, Trägersysteme zur Verfügung zu stellen, die aus Lipiden mit physiologischer Verträglichkeit bestehen. Diese Trägersysteme sollen ein hohes Aufnahmevermögen für Wirkstoffe aufweisen und sich im Laufe der Lagerung nicht substantiell verändern.

Es war somit überraschend und für den Fachmann nicht vorherzusehen, daß die Verwendung spezieller Suspensionen, die als wesentliche Bestandteile flüssige Öle aufweisen, welche durch z. B. Wachse amorph verfestigt sind, gemäß Anspruch 1 die Lösung dieser Aufgabe darstellen würde.

Bevorzugte Ausgestaltungen dieses Trägersystems sind wiederum Gegenstand der Unteransprüche.

Der Begriff Suspension bzw. Dispersion wird hier als Überbegriff in seinem weitesten Sinne verwendet und beschreibt die Verteilung einer diskontinuierlichen Phase in einer kontinuierlichen Phase. Die diskontinuierliche Phase kann dabei halbfest, teilweise fest oder fest vorliegen und ist teilkristallin bzw. überwiegend nicht-kristallin. Die kontinuierliche Phase kann flüssig, halbfest oder fest, nicht aber gasförmig, sein.

Die Ölphase des Trägersystems enthält mindestens zwei Komponenten. Der erste essentielle Bestandteil ist ein flüssiges Öl. Der Schmelzpunkt dieses Öls liegt unter 4 °C. Bevorzugte Öle sind Verbindungen von kurzkettigen (weniger als 14 Kohlenstoffatome) Fettalkoholen. Hierzu gehören unter anderem Isopropylmyristat, -palmitat, -stearate, Octyldodecanol, $C_{6-14}$ Dicarbonsäurediester des Isopropylalkohols, $C_{14-20}$ verzweigtkettige, aliphatische Fettalkohole, $C_{6-14}$ Fettsäuretriglyceride und -diglyceride, $C_{12-16}$ Oktanoate, Tridecylsalicylate und Öle der Crodamol® Gruppe.

Der zweite essentielle Bestandteil der Ölphase ist ein lipophiler, verfestigender Stoff, welcher bei 37 °C fest ist. Dieser wird insbesondere aus der Gruppe der Lipide mit einem Schmelzpunkt über 40 °C und gegebenenfalls der lipophilen Gelbildner (z. B. hydrophobe Polymere) gewählt. Geeignete Stoffe sind Ester langkettiger Fettalkohole mit langkettigen Fettsäuren, Wachse, bestimmte Glyceride und langkettige Fettalkohole, jeweils mit einem Schmelzpunkt über 40°C. Insbesondere wird dieser Stoff aus der Gruppe Carnaubawachs, Hydroxyoctacosanylhydroxystearat, chinesischer Wachs, Cetylpalmitat, Bienenwachs und ähnlicher Wachse gewählt. Weitere Beispiel für diese verfestigende Stoffe sind $C_{20-40}$ Di- und Triglyceride, auch solche die ungesättigte Fettsäuren enthalten, $C_{20-40}$ Fettalkohole, $C_{20-40}$ Fettamine und deren Verbindungen, Sterole.

Das flüssige Öl und das strukturierende Agens werden bevorzugt in einem Verhältnis von 99 + 1 bis 50 + 50, insbesondere in einem Verhältnis von 95 + 5 bis 80 + 20, gemischt. Die Mischung aus polarem Öl und strukturierendem Agens ist, nach gemeinsamen Erwärmen der Komponenten auf 90 °C und anschließendem Abkühlen unter Rühren auf Umgebungstemperatur, bei 21 °C halbfest oder fest. Bei der Mischung handelt es sich überwiegend um einen amorphen, nicht-kristallinen Feststoff oder halbfesten Stoff. Der Begriff Feststoff wird hier nach Bauer et al. (Bauer et al., Pharmazeutische Technologie, 4. Auflage, Georg Thieme Verlag Stuttgart, New York, 1993, Seite 43) wie folgt definiert:

"Feststoffe sind formstabile, unter dem Einfluß mäßiger mechanischer Kräfte elastische Körper". Halbfeste Stoffe "zeichnen sich dadurch aus, daß sie nur eine begrenzte Formstabilität besitzen" (Bauer et al., Pharmazeutische Technologie, 4. Auflage,

Georg Thieme Verlag Stuttgart, New York, 1993, Seite 253). Der amorphe Zustand wird dadurch nachgewiesen werden, daß die Mischung keine oder nur sehr schwache oder breite Röntgenreflexe im Vergleich zu der kristallinen Referenzsubstanz Cetylpalmitat aufweist. Die Abwesenheit von kristallinen Anteilen kann unter anderem durch Verwendung eines Mikroskops mit polarisiertem Licht sichtbar gemacht werden. Hierbei leuchten im allgemeine kristalline Bereiche auf, während amorphe oder flüssige Bereiche dunkel bleiben. Die Mischung sollte also unter dem Mikroskop mit polarisiertem Licht bevorzugt keine leuchtenden Bereiche aufweisen. Die Kristallinität in der Mischung kann auch mit einem dynamischen Differenz-Wärmestromkalorimeter (differential scanning calorimetry, DSC) ermittelt werden. Der Kristallinitätsindex (KI) kann als Verhältnis der Kristallinität der Rohware und der Kristallinität der Mischung der beiden Komponenten definiert werden. Die Kristallinität wird hierbei durch die Höhe des Schmelzpeaks (z. B. in mW) pro Gramm des kristallinen Lipides bestimmt. Diese Höhe des Schmelzpeaks des Rohmaterials des verfestigenden Agens wird mit "Roh" bezeichnet und die Höhe des Schmelzpeaks in der Mischung mit "Mix" bezeichnet:

$$KI = \frac{Mix}{Roh}$$

Der Kristallinitätsindex ist vorteilhaft unter 0,5.

Die Ölphase kann Mischungen der genannten Komponenten und neben den genannten zwei essentiellen Komponenten weitere lipophile Stoffe enthalten, solange die resultierende Mischung, nach gemeinsamen Erwärmen der Komponenten auf 90 °C und anschließendem Abkühlen unter Rühren auf Raumtemperatur, fest oder halbfest ist und überwiegend amorph bleibt. Beispiele weitere Komponenten sind lipophile Arzneistoffe und kosmetische Inhaltsstoffe, pflanzliche und natürliche Öle und Fette, lipophile Antioxidantien, Sonnenschutzmittel, ätherische Öle, Parfüme, Pflanzenextrakte usw.

Ein dritter essentieller Bestandteil des erfindungsgemäßen Trägersystems ist Wasser oder eine mit Wasser mischbare Flüssigkeit. In einer bevorzugten Ausführungsform der vorliegenden Erfindung enthält die Wasserphase einen gelbildenden, strukturierenden Zusatz, wodurch die Wasserphase halbfest wird und über eine Fließgrenze von 5 Pa oder darüber bei 21 °C verfügt (ermittelt z. B. mit einem Rheometer). Geeignete strukturierende Zusätze sind hydrophile Polymere, gewisse anorganische Gelbildner und amphiphile Stoffe. Beispiele für Polymere sind Alginate, Zellulosederivate, Xanthan Gummi, Stärke und -derivate. Beispiele für anorganische Gelbildner sind Aerosil Typen und Bentonite. Beispiele für amphiphile Stoffe mit viskositätserhöhender Wirkung sind Glycerolmonostearat und Poloxamer 127. Bevorzugte strukturierende Agenzien sind polyelektrolytische Polymere wie z. B. Polyacrylsäure, Carboxymethylcellulose oder Carrageenan.

Die Wasserphase kann weitere Zusätze enthalten wie z. B. hydrophile oder amphiphile Arzneistoffe oder kosmetische Inhaltsstoffe, wasserlösliche Antioxidantien, Konservierungmittel, Feuchthaltemittel oder Pflanzenextrakte.

Desweiteren enthält die Suspension notwendigerweise Stoffe, welche die physikalische Stabilität der Suspension erhöhen. Dies können die bereits oben genannten gelbildende Polymere oder amphiphile Substanzen (Emulgatoren) sein. Geeignete Emulgatoren sind Myristyl-, Cetyl-, Stearylalkohol, Polysorbate, Sorbitanester, Blockpolymere (z. B. Poloxamere), Glycerolmonofettsäureester (z. B. Glycerolmonostearat), Ester von Polycarbonsäuren und Fettalkohole, Mono- und Diglyceride von Fettsäuren verestert mit Milchsäure, Zitronensäure oder Weinsäure (z. B. Glycerolstearatcitrat). Vorteilhaft ist die Verwendung einer Kombination aus mindestens zwei Emulgatoren. Dabei sollte ein Emulgator geladen sein (positiv, negativ oder ampholytisch). Beispiele hierfür sind Glycerolstearatcitrat und quartäre Ammoniumverbindungen (z. B. Cetylpyridiniumchlorid).

Die Ölphase, Wasserphase und die Suspensionsstabilisatoren werden gemischt, um eine innige Verteilung der Ölphase in der kontinuierlichen Wasserphase zu erzielen. Die Größe der Öltröpfchen liegt typischerweise zwischen 1 $\mu$m und 100 $\mu$m. Die kontinuierliche Wasserphase der Suspension kann z. B. durch ein rasches Lösungsvermögen für hydrophile Farbstoffe oder durch die Mischbarkeit mit Wasser charakterisiert werden. Der Anteil der Wasserphase an der gesamten Suspension liegt vorteilhaft bei 40 - 95 %.

Die Lipidpartikel mit einem großen oder überwiegend flüssigen Anteil in der Lipidmischung (i.d.R. > 50%) werden an den Beispielen 21 bis 26 näher erläutert.

Durch kontrolliert induzierten Übergang (Kristallisation) in die stabilen $\beta$-Modifikationen von Lipiden kann der Wirkstoff nach Wunsch freigesetzt werden (Beispiele 14-16). Reize zur Auslösung dieses Übergangs sind Zugabe von Elektrolyten, Temperaturerhöhung oder Entzug von Wasser aus der NCC Dispersion.

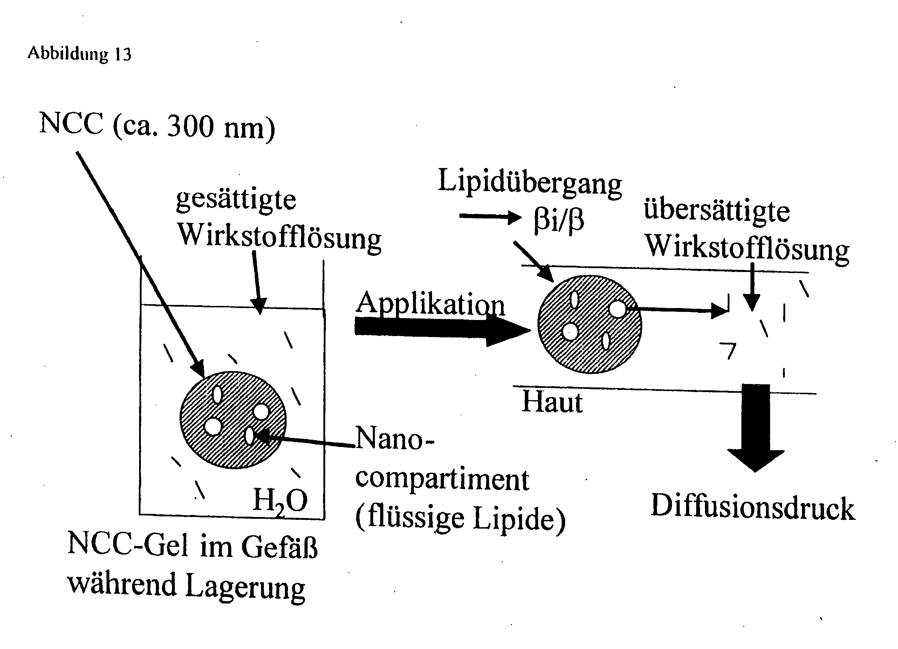

Ein Beispiel für Wasserentzug ist das Trocknen von Partikeldispersionen nach topischer Applikation auf der Haut. Induktion von Modifikationsübergang und Wirkstofffreisetzung kann bei ausreichend sensitiven Systemen bereits durch die auf der Haut vorhandenen Elektrolyte erfolgen. Dies ist von besonderem Interesse für Wirkstoffe wie Cyclosporin (Beispiel 6), die nach topischer Applikation eine zu geringe Penetration in die Haut haben um z.B. Psoriasis erfolgreich therapieren zu können. Im Vergleich zu anderen partikulären Trägern, bei denen die Freisetzung auf reiner Diffusion basiert, wird bei NCC der Wirkstoff aktiv aus dem Carrier transportiert. Treibende Kraft ist die induzierte Bildung von perfekt kristallinen Partikeln, in denen kein Platz mehr für Wirkstoffmoleküle ist. Der Arzneistoff (z.B. Cyclosporin) wird in die äußere Phase gedrängt (z.B. Wasser der NCC-haltigen Lotion oder Creme), in der er gering löslich ist. Als Folge kommt es zu einer Übersättigung der Wasserphase mit Arzneistoff und es entsteht folglich ein erhöhter Diffusionsdruck des Arzneistoffes in die Haut (= Erhöhung der thermodynamischen Aktivität des Wirkstoffes) (Abb. 13). Somit eignen sich die NCC insbesondere für Wirkstoffe mit Bioverfügbarkeitsproblemen, z.B. Cyclosporine (z.B. Cyclosporin A) und strukturell verwandte Moleküle. Als besonders geeignet für Cyclosporine erwiesen sich Mischungen aus festen Lipiden (z.B. Imwitor und Compritol) und flüssigen Lipiden (Ölen (z.B. Rizinusöl, Olivenöl, Maiskeimöl, Softigen, Isopropylmyristat, Octyldodecanol und Miglyole)).

Die erfindungsgemäßen Partikel können in hoher Feststoffkonzentration als Dispersion hergestellt werden. Somit vermeidet man die oben beschriebenen Nachteile bei der Verarbeitung bisheriger, niedrig konzentrierter Lipidpartikeldispersionen in andere Arzneiformen wie z.B. topische Arzneiformen und Kosmetika (Cremes), orale Arzneiformen (wie z.B. Tabletten, Pellets und Kapseln) sowie bei Parenteralia. Lipidpartikeldispersionen können nicht nur in Tabletten, Filmtabletten und Dragess eingearbeitet sondern auch auf diese aufgezogen werden. Hierzu werden die Tabletten oder Dragees mit Partikeldispersion besprüht (z.B. in einem Kugel-Coater (Glatt), Wurster Apparatur, Fluid Bed Dryer, Accela Cota) oder die Lipidpartikeldispersion wird in einem Dragierkessel zugegeben. Unbeladene Lipidpartikel können benutzt werden, um einen Schutzfilm zu erzeugen (z.B. gegen Sauerstoff und Luftfeuchtigkeit), einen Film zur Modulation der Arzneistoffliberation oder zum Polieren von Dragess und Filmtabletten. Wirkstoffbeladene Partikel könne eine separate Arzneistoffdosis kontrolliert freisetzen, z.B. Initialdosis.

Polieren von Dragees und Filmtabletten erfolgt bisher durch Zugabe von Wachskugeln (z.B. Carnaubawachs) oder durch Aufsprühen von Wachsen in organischen Lösungsmitteln. Verwendung von Lipidpartikeldispersionen (Partikelgröße Mikro- bzw, Nanometer) ergibt im Vergleich zu Wachskugeln (Durchmesser z.B. 1 cm) eine feinere Verteilung des Lipids auf der Drageeoberfläche und vermeidet organische Lösungsmittel. Niedrig konzentrierte Lipidpartikeldispersionen sind zu diesem Zweck nicht einsetzbar, da aufgrund des hohen Wassergehaltes ein Anlösen z.B. der Drageeoberfläche eintreten kann. Die erfindungsgemäßen Partikeldispersionen zeichnen sich dagegen durch ihren relativ geringen Wasseranteil aus.

Bei Herstellung von Lipidpartikel in nicht-wäßrigen, bevorzugt öligen Medien und flüssigen Polyethylenglykolen (z.B. PEG 400 und PEG 600), können die Dispersionen direkt in Weich- oder Hartgelatinekapseln abgefüllt werden. Bei Herstellung in bei Raumtemperatur festem PEG (z.B. PEG 6000) kann das erstarrte Produkt (Dispersion von Lipidpartikeln in festem PEG) gemahlen und als Pulver in Hartgelatinekapseln abgefüllt werden.

Beispiele

Beispiel 1: Herstellung einer 40 % Lipidpartikeldispersion (Feststoffgehalt 45 %):

(nicht zur Erfindung gehörig)

Die Zusammensetzung der Lipidpartikel-Dispersion war 40 % Cetylpalmitat, 5 % Saccharoseester S-1670 (Mitsubishi-Kagaku Foods Corporation, Tokyo, Japan) und Wasser auf 100 %. Lipid wurde auf 90 °C erhitzt und mit einem Rotor-Stator-Rührer (Ultra-Turrax, Janke & Kunkel, Deutschland) bei 8000 Umdrehungen pro Minute für 2 Minuten mit der heißen wäßrigen Lösung des Tensids gemischt. Die erhaltene Rohemulsion wurde dann in einem Micron LAB 40 bei 500 bar und 3 Zyklen bei 80 °C homogenisiert. Das Produkt war weiß und von cremiger Konsistenz. Nach Abkühlung und Kristallisation der Lipidnanopartikel wurde die Teilchengröße im Produkt ermittelt. Der Durchmesser betrug 246 nm, der Polydispersitätsindex 0,179 (Meßmethode: Photonenkorrelationsspektroskopie (PCS), Gerät: Zetasizer 4, Malvern Instruments, UK).

Beispiel 2: Herstellung einer 50 % Lipidpartikeldispersion (Feststoffgehalt 55 %):

(nicht zur Erfindung gehörig)

Die Rezeptur von Beispiel 1 wurde verwendet, wobei der Lipidanteil von 40 % auf 50 % erhöht wurde. Herstellung und Homogenisation erfolgte wie in Beispiel 1. Das erhaltene Produkt wurde zur Bestimmung der Partikelgröße verdünnt, der PCS-Durchmesser betrug 325 Nanometer, der Polydispersitätsindex 0,190.

Beispiel 3: Herstellung einer 40 % Lipidpartikeldispersion (Feststoffgehalt 45 %) mit einem Rotor-Stator-Rührer:

(nicht zur Erfindung gehörig)

Zusammensetzung der Lipidpartikel-Dispersion: 40 % Fett, 5 % Saccharoseester S-1670 (Mitsubishi-Kagaku Foods Corporation, Tokyo, Japan) und Wasser auf 100 %. Es wurden 24 g wäßrige Tensidlösung auf 80 °C erhitzt, 4 g geschmolzenes Lipid zugegeben und für 2 Minuten mit einem Ultra-Turrax (Janke & Kunkel, Deutschland) bei 8000 Umdrehungen pro Minute für 2 Minuten dispergiert. Anschließend wurden erneut 4 g geschmolzenes Lipid zugegeben, Dispergierbedingungen wie vorher. Es erfolgte eine sukzessive Zugabe von jeweils 4 g geschmolzenes Lipid, bis der gesamte Lipidgehalt 40 % betrug. Nach Abkühlen und Kristallisation der Lipidpartikel wurde eine Partikelgrößenmessung in Wasser durchgeführt. Der Durchmesser 50 % betrug 12,25 $\mu$m (Meßmethode: Laserdiffraktometrie, Gerät: Mastersizer E, Malvern Instruments, UK). Gemessen wurde eine Volumenverteilungskurve.

Beispiel 4: Herstellung einer 70 % Lipidpartikeldispersion (Feststoffgehalt 75 %):

(nicht zur Erfindung gehörig)

Es wurde die Rezeptur aus Beispiel 3 mit 5 % Saccharosester verwandt, der Lipidgehalt wurde jedoch von 40 % auf 70 % erhöht. Herstellung erfolgte wie in Beispiel 3, wobei jeweils sukzessive 4 g Lipid zugegeben wurden, bis die maximale Lipidkonzentration 70 % betrug. Nach Abkühlung wurde ein Durchmesser 50 % von 20,68 $\mu$m gemessen (Laserdiffraktometrie wie Beispiel 3).

Beispiel 5: Lagerstabilität der hochkonzentrierten Lipidpartikeldispersionen:

(nicht zur Erfindung gehörig)

Die Dispersion aus Beispiel 1 wurde bei Raumtemperatur 14 Tage gelagert. Bestimmung der Partikelgröße mit PCS ergab einen Durchmesser von 243 nm und einen Polydispersitätsindex von 0,203. Es kam zu keinem signifikanten Partikelgrößenanstieg, die Dispersion ist in der hochkonzentrierten Form physikalisch stabil.

Beispiel 6: Herstellung einer arzneistoffhaltigen 50 % Lipidpartikeldispersion (Feststoffgehalt 55 %):

(nicht zur Erfindung gehörig)

Die Zusammensetzung der Lipidpartikeldispersion war 48 % Imwitor 900, 2 % Cyclosporin A, 5 % Tween 80 und Wasser auf 100 %. Lipid und Arzneistoff wurden auf 90 °C erhitzt und mit einem Rotor-Stator-Rührer (Ultra-Turrax, Janke & Kunkel, Deutschland) bei 8000 Umdrehungen pro Minute für 2 Minuten mit der heißen wäßrigen Lösung des Tensids gemischt. Die erhaltene Rohemulsion wurde dann in einem Micron LAB 40 bei 500 bar und 3 Zyklen bei 80 °C homogenisiert.

Beipiel 7:

(nicht zur Erfindung gehörig)

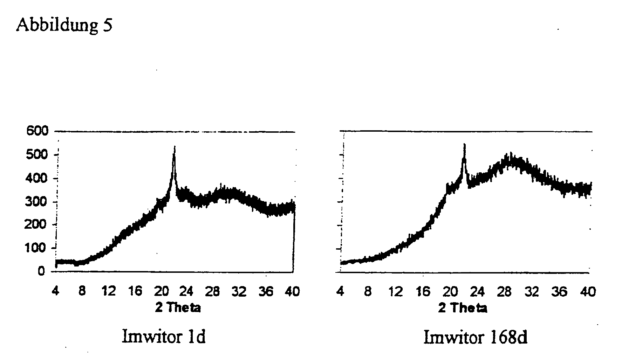

Die Cyclosporin-beladene Lipidpartikeldispersion wurde mit Differential Scanning Calorimetry (DSC) auf kristallinen Status untersucht. Die Messungen zeigen, daß die Lipidpartikel überwiegend in $\alpha$-Modifikation vorliegen (Onset-Temperatur 51,5 °C, Peakmaximum 58,7 °C) während das reine Lipid vorwiegend $\beta$-Modifikation besitzt (Onset-Temperatur 54,2 °C, Peakmaximum 61,9 °C) (Abb. 5).

Beispiel 8: Herstellung einer 80 % Lipidpartikeldispersion (Feststoffgehalt 85%):

(nicht zur Erfindung gehörig)

Die Rezeptur von Beispiel 1 wurde verwendet, wobei der Lipidanteil von 40 % auf 80 % erhöht wurde. Zuerst wurde eine 50 % Lipidpartikeldispersion analog Beispiel 1 hergestellt. Zum erhaltenen Produkt wurde sukzessive in Teilschritten jeweils 10 % Lipid unter Rühren mittels eines Rotor-Stator-Rührers bei 8000 Umdrehungen pro Minute bis zum Erhalt einer 80 % Lipidpartikel-Dispersion zugegeben. Nach Abkühlen wurde ein Durchmesser 99 % von 77,66 $\mu$m gemessen (Laserdiffraktometrie wie Beispiel 3).

Beispiel 9: Vernebelung einer 10 % Lipidpartikeldispersion mittels eines Pariboy (Paul Ritzau Pari-Werk GmbH, Starnberg, Deutschland):

(nicht zur Erfindung gehörig)

Die Zusammensetzung der Lipidpartikel-Dispersion war 10 % Cetylpalmitat, 1,2 % Tego Care 450 und Wasser auf 100 %. Herstellung und Homogenisation erfolgte wie in Beispiel 1. Das erhaltene Produkt wurde mittels Pariboy vernebelt und das anfallende Aerosol in einem Becherglas aufgefangen. Der Durchmesser 50 % betrug 0,28 $\mu$m vor und 0,30 $\mu$m nach Vernebelung (Laserdiffraktometrie wie Beispiel 3).

Beispiel 10: Herstellung von flüssig-fest Partikeln mit Imwitor:

Die Zusammensetzung betrug 10 % Imwitor, 5 % Miglyol 812, 0,5 % Retinol, 2,5 % Miranol (Natriumcocoamphoacetat) und 82 % Wasser. Die Lipide Imwitor und Miglyol wurden im geschmolzenen Zustand bei 90 °C gemischt und dann Partikel wie in Beispiel 1 hergestellt. Der PCS-Durchmesser betrug 188 nm, der Polydispersitätsindex 0,266. Das Weitwinkelröntgendiffraktogramm (Abb. 5, links) belegt das überwiegende Vorliegen des Fettes im flüssigen Zustand ($\alpha$-Modifikation).

Beispiel 11: Herstellung von flüssig-fest Partikeln mit Compritol:

Die Herstellung erfolgte wie in Beispiel 10, das Lipid Imwitor wurde durch Compritol ersetzt. Die Konzentration an Miranol betrug hier 1,5 %. Der PCS-Durchmesser betrug 225 nm, der Polydispersitätsindex 0,205.

Beispiel 12: Herstellung von flüssig-fest Partikeln mit unterschiedlichem Anteil an flüssigem Lipid:

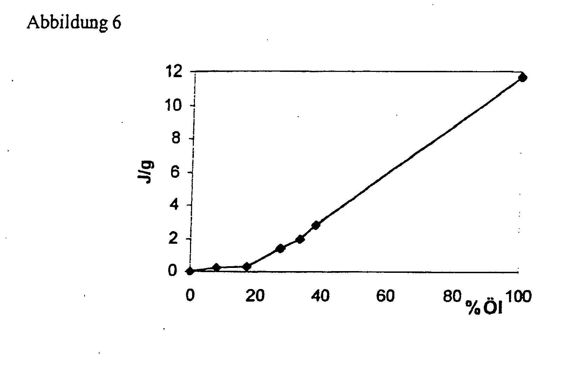

Es wurden Compritol-Partikel wie in Beispiel 11 beschrieben 5 hergestellt. Der Lipidanteil betrug konstant 15 % (festes Lipid Compritol + Öl Miglyol) in der wäßrigen Dispersion, wobei das Verhältnis Öl zu festem Lipid verändert wurde. Es wurden Partikel hergestellt mit 8,3 %, 16,7 %, 28 % und 38 % Ölanteil im Lipid (d.h. 91,7 %, 83,3 %, 72 % und 62 % Compritol). Bei niedrigem Ölanteil (8,3 %) in der Mischung löst sich das Öl überwiegend im festen Lipid (molekulardisperse Verteilung), es entstehen nur wenige Nanokompartimente aus flüssigem Öl. Molekulardispers verteiltes Öl kann nicht kristallisieren, Kristallisation und Freisetzung von Kristallgitterenergie tritt nur auf bei einer ausreichend großen Ansammlung von Molekülen (z.B. Nanokompartiment), daher ist die gemessene Schmelzenthalpie berechnet auf die insgesamt eingearbeitete Menge Öl weit unter dem theoretischen Wert von 12 J/g und nahe Null (Abb. 6, links). Bei Erhöhung des Ölanteils erreicht man die Grenze der Aufnahmefähigkeit der festen Lipidmatrix für Ölmoleküle, es bilden sich vorwiegend kleine Bereiche mit flüssigem Öl. Diese Nanokompartimente können kristallisieren und die Enthalpie steigt linear von 16,7 % bis 38 % Ölanteil an (Abb. 6).

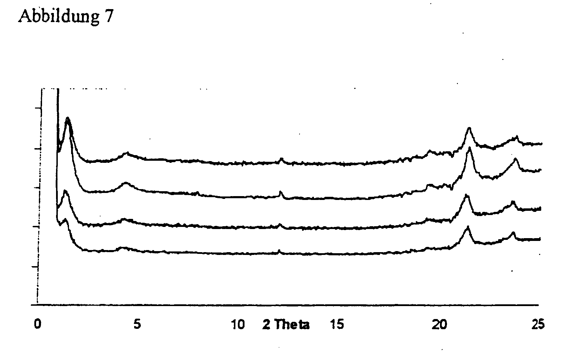

Die Röntgendiffraktogramme der Partikel belegen, daß neben dem mit DSC nachgewiesenem flüssigen Lipid in $\alpha$-Modifikation auch festes Lipid in der instabilen $\beta$'-Modifikation vorliegt (Peaks bei 0,38 nm und 0,42 nm spacing, Abb. 7).

Beispiel 13: Konservierung des flüssigen Anteils in Lipidpartikeln durch Hemmung der Transformation des Lipids in die stabile $\beta$-Modifikation:

20 Teile wäßrige SLN-Dispersion aus Beispiel 10 wurden durch Rühren in 80 Teile einer kosmetischen O/W Creme eingearbeitet (Nivea Visage, Firma Beiersdorf, Hamburg, Germany). Nach 168 Tagen Lagerung bei Raumtemperatur zeigte das Röntgendiffraktogramm im Vergleich zum Tag nach der Herstellung keine Änderung (Abb. 5).

Beispiel 14: Kontrollierte Kristallisation des flüssigen Anteils und Überführung von $\alpha$/$\beta$' in die stabile $\beta i$/$\beta$-Modifikation durch Elektrolyte:

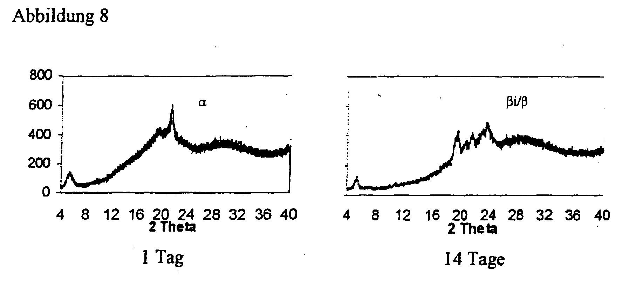

Zu 20 Teilen der Imwitor Partikel aus Beispiel 10 werden 10 Teile Glycerol und 70 Teile Wasser zugesetzt. Zu dieser Mischung werden 0,4 % Carbopol 940 (Polyacrylsäure) als Gelbildner zugegeben und anschließend als Elektrolyt 0,1 % Natronhydroxid zugesetzt. Direkt nach Herstellung zeigen die flüssig-festen Lipidpartikel noch unverändert einen ausgeprägten flüssigen Anteil $\alpha$-Modifikation (Abb. 8, links), der bei Einwirkung von Elektrolyt zu einem festen Lipidpartikel aus der stabilen $\beta i$/$\beta$-Modifikation wird (Abb. 8, rechts).

Beispiel 15: Kontrollierte Überführung von $\alpha$/$\beta$' in die stabile $\beta i$/$\beta$-Modifikation durch Wasserentzug:

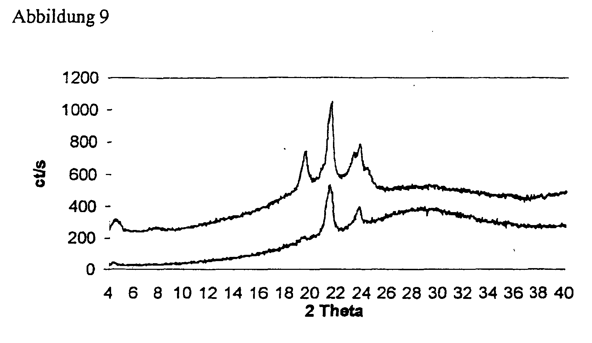

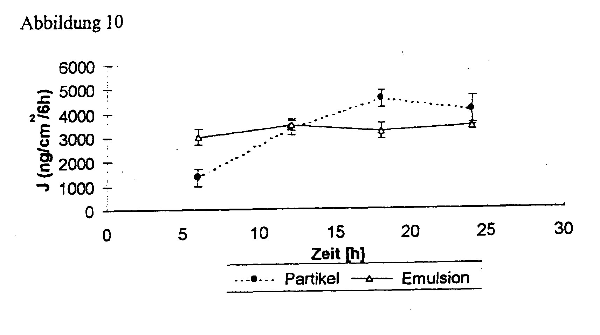

Eine Compritol-Partikeldispersion wurde wie in Beispiel 11 hergestellt, wobei das Miglyol 10 % Retinol enthielt. Zur Untersuchung der Freisetzung wurden 200 $\mu$l wäßrige SLN Dispersion in eine Durchfluß-Franz-Zelle (Crown Scientific, US-Sommerville) eingebracht (Akzeptormedium: isotonischer Phosphatpuffer pH 7,4, Flußrate Medium: 1,0 ml/h, Temperatur: 37 °C, Membran: Cellulosenitratfilter getränkt mit Isopropylmyristat). Die Röntgendiffraktogramme zeigen daß die Verdunstung von Wasser zu einer Ausbildung eines festen Lipidpartikels mit stabiler $\beta i$-Modifikation führt (Abb. 9, Peak bei 0,46 nm spacing). Mit zunehmender Überführung in ein festes Partikel mit weniger Kristallfehlstellen wird zunehmend mehr Arzneistoff aus dem Partikel verdrängt, das heißt die Freisetzung (Arzneistoff-Flux) nimmt mit der Zeit (= zunehmendem Kristallisationsgrad) zu (Abb.10). Zum Vergleich wurde eine Nanoemulsion mit vergleichbarer Tropfen größe hergestellt, indem Compritol im Herstellungsgang durch Miglyol ersetzt wurde (PCS-Durchmesser: 186 nm, PI 0,113).

Beispiel 16:

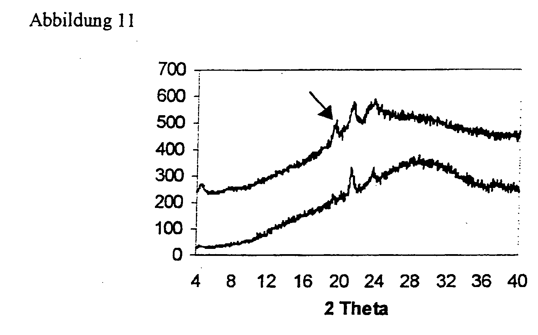

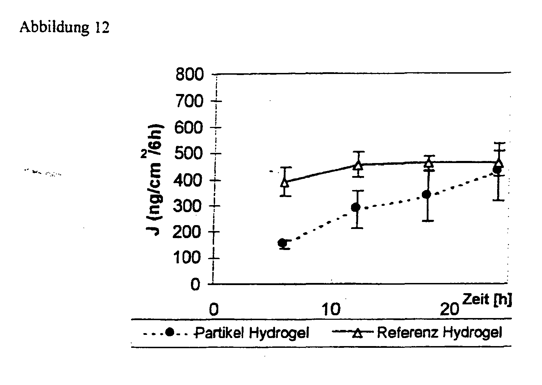

Mit Retinol-beladene Partikel wurden wie in Beispiel 15 beschrieben hergestellt. Zu 20 Teilen der Partikeldispersion wurden 10 Teile Glycerol und 70 Teile Wasser zugesetzt. Zu dieser Mischung wurden 0,5 % Xanthan Gum zugegeben. Das gebildete Hydrogel wurde wie in Beispiel 15 in der Franz-Zelle untersucht. Durch Wasserverdunstung bildete sich auch im Hydrogel die stabilere $\beta i$-Modifikation (Abb. 11), der Arzneistoff wurde zunehmend stärker aus der Lipidmatrix verdrängt und der Flux (Arzneistofffreisetzung) stieg mit der Zeit an (Abb. 12).

Beispiel 17: Herstellung einer Dispersion zum Polieren von Dragees:

(nicht zur Erfindung gehörig)

Lipidpartikel auf der Basis von Carnaubawachs wurden durch Hochdruckhomogenisation hergestellt (31 % Carnaubawachs, 3 % Tensid, Wasser). Herstellung erfolgte mit einem Micron LAB 40 bei 95 °C, 500 bar und 3 Zyklen. Der PCS-Durchmesser betrug 420 nm, der Polydispersitätsindex lag bei 0,185.

Beispiel 18: Prolongierte Wirkstofffreisetzung von Zitronenöl aus Stearinsäurepartikeln im Vergleich zu Miglyol-Emulsionen:

(nicht zur Erfindung gehörig)

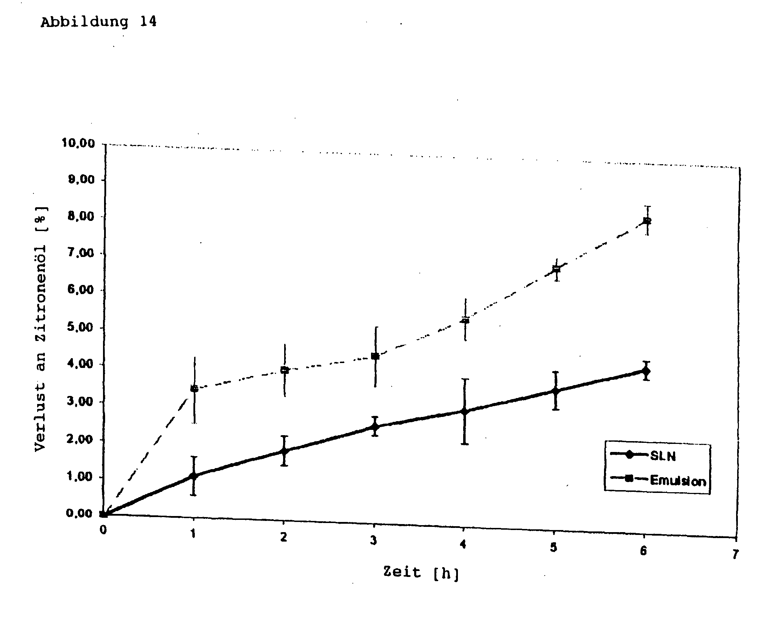

Es wurde eine Lipidpartikeldispersion bestehend aus 10 % (m/m) Stearinsäure, 1% (m/m) Zitronenöl, 1,2 % (m/m) Tween 80 und Wasser durch Hochdruckhomogenisation hergestellt. Die Mischung von Lipid und Emulgator wurde bei 75 °C geschmolzen und in der wäßrigen Lösung mit einem Ultra-Turrax T25 mit Dispergierwerkzeug S25, Janke und Kunkel, dispergiert (8000 rpm, für 1 Minute). Die erhaltene Rohemulsion wurde dann mit einem APV Gaulin LAB 40 Homogenisator bei 500 bar mit 3 Zyklen bei 75 °C homogenisiert. Es entstanden Lipidpartikel mit einem mittleren LD-Durchmesser (LD d50 %) von 215 nm. Als Vergleich wurde analog ein Emulsionssystem hergestellt, wobei die 10 % Stearinsäure durch 10 % Miglyol 812 ersetzt wurden. Der LD d50 % betrug hier 195 nm. In vitro Freisetzungsversuche bei 32 °C, die UV-spektrophotometrisch ausgewertet wurden, belegten eine verzögerte Freisetzung aus der SLN-Dispersion (kein *burst release* wie bei der Emulsion). Nach 6 Stunden war die kumulierte Freisetzung aus der SLN-Dispersion im Vergleich zur Emulsion um 50 % verringert. (Abb. 14).

Beispiel 19: Prolongierte Wirkstofffreisetzung des Parfumöls Allure (Chanel) aus Stearinsäurepartikeln im Vergleich zu Miglyol-Emulsionen:

(nicht zur Erfindung gehörig)

Es wurde eine Lipidpartikeldispersion bestehend aus 10 % (m/m) Stearinsäure, 1% (m/m) Allure, 1,2 % (m/m) Tween® 80, und Wasser durch Hochdruckhomogenisation analog Beispiel 18 hergestellt. Es entstanden Lipidpartikel mit einem mittleren PCS-Durchmesser von 336 nm und einem Polydispersitätsindex von 0,137. Als Vergleich wurde analog ein Emulsionssystem hergestellt, wobei die 10 % Stearinsäure durch 10 % Miglyol 812 ersetzt wurden. In vitro Freisetzungsversuche bei 32 C, die UV-spektrophotometrisch ausgewertet wurden, belegten eine verlängerte Freisetzung aus der SLN-Dispersion. Nach 6 Stunden waren aus dem Emulsionssystem bereits 100 % des Parfumöls freigesetzt; die Freisetzung aus der SLN-Dispersion betrug zu diesem Zeitpunkt jedoch lediglich 75 %.

Beispiel 20:

(nicht zur Erfindung gehörig)

Es wurden Lipidpartikeldispersionen mit Witepsol H15 (Hartfett $C_{12}$-$C_{18}$) und Witepsol E85 (Hartfett $C_{12}$-$C_{18}$) durch Hochdruckhomogenisation hergestellt (drei Zyklen, 500 bar, Herstellungstemperatur 85°C, Gerät LAB 40). Die Partikelgröße betrugen 119 nm und 133 nm (PCS-Durchmesser). Die Lipidpartikel wurden mit einem Bruker mini-spec pc 120 auf die Veränderung der Relaxationszeiten T1 und T2 untersucht. Die Relaxationszeiten betrugen T1 = 0,1498 s und T2 = 0,0707 s (Witepsol E85) und T1 = 0,1577 s und T2 = 0,1191 s (Witepsol H15).

Beispiel 21:

Dieses Beispiel zeigt in 1 die Zusammensetzung einer typischen Suspension, die den Ansprüchen dieser Erfindung genügt. Als Vergleichsbeispiel ist die Rezeptur V1 angegeben, die nicht den Ansprüchen dieser Erfindung genügt.

| | 1 | V1 |
|---|---|---|
| Vaseline | - | 5,0 % |
| Paraffinöl | - | 10,0 % |
| Dimeticon 100 | - | 5,0 % |
| Jojobaöl | - | 4,0 |
| Isopropylmyristat | 10,0 % | - |
| Mittelkettige Triglyceride | 10,0 % | - |
| Hydroxyoctacosanylhydroxystearat | 4,0 % | - |
| Retinaldehyd | 0,2 % | 0,2 % |
| Glycerolmonostearat | 0,3 % | 0,3 % |
| Cetylalkohol | 0,5 % | 0,5 % |

(fortgesetzt)

| | 1 | V1 |
|---|---|---|
| Polysorbat 80 | 0,5 % | 0,5 % |
| Glycerolstearatcitrat | 2,0 % | 2,0 % |
| Sorbitol | 5,0 % | 5,0 % |
| Polyacrylsäure | 0,5 % | 0,5 % |
| Natriumhydroxid | Zur Einstellung des pH-Werts (pH 6 - 7) | Zur Einstellung des pH-Werts (pH 6 - 7) |
| Natriumchlorid | 0,5 % | 0,5 % |
| Wasser | ad 100 % | ad 100 % |

Die lipophilen Komponenten, einschließlich des Wirkstoffs, und die hydrophilen Komponenten, einschließlich des Emulgators aber ohne die Polyacrylsäure, werden getrennt auf 90 °C erhitzt und bei 90 °C gemischt. Die Mischung wird in der Hitze 5 Minuten mit einem Ultra-Turrax bei 10.000 Umdrehungen pro Minute dispergiert. Nach Abkühlen unter Rühren auf 40 °C wird die Polyacrylsäure zugegeben, nochmals 1 Minute mit dem Ultra-Turrax dispergiert und anschließend die Suspension unter Rühren auf Umgebungstemperatur abgekühlt.

Beispiel 22:

Dieses Beispiel zeigt die Möglichkeit auf, durch die Erfindung instabile Wirkstoffe zu stabilisieren. Die Vergleichsrezeptur V1 zeigt keine amorph verfestigte Ölphase auf und ist daher nicht erfindungsgemäß zusammengesetzt. Die Stabilitäten der beiden retinaldehydhaltigen Zubereitungen aus Beispiel 21 sind in der Tabelle 1 aufgelistet. Die Lagerung erfolgt bei Raumtemperatur (RT) oder 40 °C in geschlossenen Glasgefäßen, die 1/3 der Zubereitung und 2/3 Luft enthalten. Die Werte sind in Prozent der Ausgangskonzentration ausgedrückt. Die Gehaltsbestimmungen wurden mit UV-Spektroskopie bei einer Detektionswellenlänge von 325 nm vorgenommen.

Tabelle 1

| Zeit (d) | 14 Tage | | 28 Tage | | 84 Tage | |
|---|---|---|---|---|---|---|
| | RT | 40 °C | RT | 40 °C | RT | 40 °C |
| 1 | 92,6 | 75,1 | 89,2 | 64,2 | 82,8 | 35,7 |
| V1 | 85,9 | 62,8 | 76,7 | 35,2 | 60,1 | 9,0 |

Die Tabelle 1 zeigt deutlich, daß nach 12 Wochen Lagerung bei 40°C für das erfindungsgemäße Trägersystem eine vielfach höhere Menge des Wirkstoffs wiedergefunden wurde als in einer Vergleichsemulsion.

Beispiel 23:

Dieses Beispiel zeigt die Möglichkeit der Reduzierung der Reizwirkung eingeschlossener Wirkstoffe auf. Der eingesetzte Wirkstoff Benzylnikotinat ruft eine Hyperämie der Haut hervor, die sich durch eine Rötung der betroffenen Hautareale erkenntlich macht. Auf zwei 2 x 2 cm großen Hautareal wurde je 8 mg der Zubereitung 2 und V2 drucklos gleichmäßig verteilt. In bestimmten Zeitintervallen wurde die Rötung der beiden Areale verglichen und auf einer Skala von 0 (keine Hautrötung) bis 4 (sehr starke Hautrötung) beurteilt. Die Zubereitungen 2 und V2 waren wie folgt zusammengesetzt:

| | 2 | V2 |
|---|---|---|
| Isopropylmyristat | 10,0 % | 10.0 % |
| Mittelkettige Triglyceride | 10.0 % | 10.0 % |
| Hydroxyoctacosanyl-hydroxystearat | 5,0 % | - |
| Jojobaöl | - | 5.0 % |
| Benzylnikotinat | 2,5 % | 2.5 % |
| Glycerolmonostearat | 0,3 % | 0.3 % |
| Cetylalkohol | 0,5 % | 0.5 % |
| Polysorbate 80 | 0,5 % | 0.5 % |
| Glycerolstearatcitrat | 2.0 % | 2.0 % |
| Sorbitol | 5,0 % | 5.0 % |

(fortgesetzt)

| | 2 | V2 |
|---|---|---|
| Polyacrylsäure | 0.8 % | 0.8 % |
| Natriumhydroxid | Zur Einstellung des pH-Werts (pH 7-8) | Zur Einstellung des pH-Werts (pH 7 - 8) |
| Natriumchlorid | 0.5 % | 0,5 % |
| Wasser | Ad 100 % | ad 100 % |

Zubereitung 2 ist erfindungsgemäß zusammengesetzt und weist eine verfestigte Ölphase auf, sodaß der Wirkstoff besser immobilisiert ist. Zubereitung V2 ist nicht erfindungsgemäß zusammengesetzt. Das feste Wachs Hydroxyoctacosanylhydroxystearat der Zubereitung 2 ist in V2 durch das flüssige Wachs Jojobaöl ersetzt wurden, sodaß die Ölphase vollständig flüssig bleibt und folglich den Wirkstoff nicht immobilisieren kann.

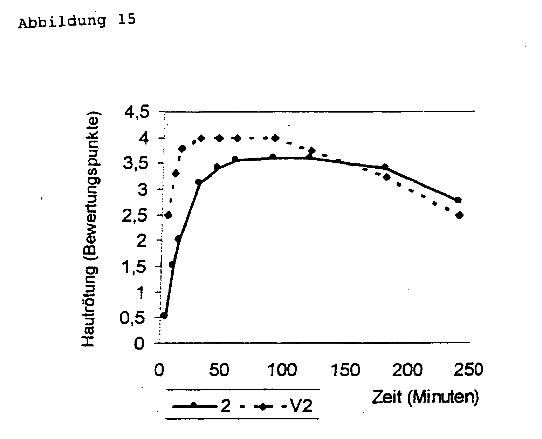

Der Verlauf der Hautrötung als Funktion der Zeit ist in der Abb. 15 dargestellt. Es sind die arithmetischen Mittelwerte von 4 Personen (2 männlich, 2 weiblich, jeweils linker Unterarm, keine Hautkrankheiten) aufgetragen. Die Abb. 15 zeigt, daß bei der erfindungsgemäßen Emulsion 2 (durchgezogene Linie) die Rötung langsamer einsetzt und die Spitze des Verlaufs geglättet ist im Vergleich zu V2 (gestrichelte Linie). Dies bedeutet eine Reduzierung der Reizwirkung des Wirkstoffs durch dessen Einschluß in das Trägersystem.

Beispiel 24:

Dieses Beispiel zeigt eine Möglichkeit zum Nachweis des Feststoffcharakters der Öltröpfchen in der Suspension auf. Die Mischung aus verfestigendem Wachs und flüssigen Öl muß, wie oben beschrieben, ein amorpher Feststoff oder halbfester Stoff sein. Der Nachweis der Formstabilität, wie oben definiert, kann bei der groben Mischung makroskopisch erfolgen. Im Nachfolgenden soll eine mögliche Methode beschrieben werden, die den Feststoffcharakter der teilweise nur wenige Mikrometer großen Öltröpfchen in der Suspension nachweisen kann. Hierbei handelt es sich um die Protonenresonanzspektroskopie ($^1$H-NMR). Diese kann die Beweglichkeit der Fettmoleküle messen. Sehr bewegliche Moleküle liefern sehr intensive und scharfe Resonanzsignale. Sehr unbewegliche Moleküle liefern hingegen nur Resonanzsignale von schwacher Intensität und großer Signalbreite (Rücker et al., Instrumentelle pharmazeutische Analytik, 2. Auflage, Wissenschaftliche Verlagsgesellschaft mbH Stuttgart, Seite 172, 1992). Bei dieser Methode wird das erfindungsgemäße Trägersystem verglichen mit einer nicht-erfindungsgemäßen Emulsion (Vergleichsemulsion), in welcher der verfestigende Stoff durch eine entsprechende Menge des flüssigen Wachs Jojobaöl ersetzt wird. Zur Auswertung werden die Signale zwischen 0,8 und 2,6 ppm herangezogen, die den Signalen der Protonen der Fettsäureketten entsprechen, insbesondere das stärkste Signal bei ca. 1,25 ppm. Die Intensität ist die Höhe dieser Signale und die Linienbreite ist die in halber Höhe des Signals gemessenen Breite. Die erfindungsgemäße Suspension zeigt die weniger intensiven und breiteren Signale im Vergleich zur Vergleichsformulierung. Die Verfestigung der Öltröpfchen zeigt sich insbesondere durch:

- die Linienbreite eines Protonensignals des Trägersystems ist mindestens doppelt so breit wie die Linienbreite des entsprechenden Signals der Vergleichsemulsion und/oder
- die Intensität eines Signals des Trägersystems ist maximal halb so groß wie die Intensität des entsprechenden Signals der Vergleichsemulsion.

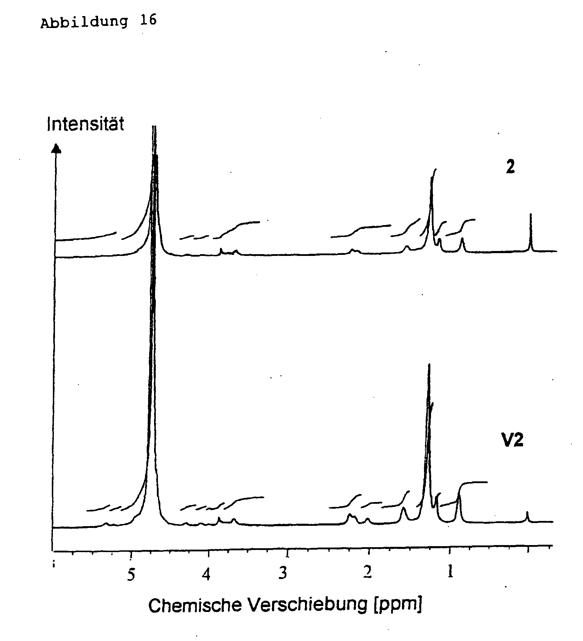

In der Abbildung 16 sind die $^1$H-NMR Spektren der Suspension 2 (oberes Spektrum) und der Vergleichsformulierung V2 (unteres Spektrum) aus Beispiel 23 dargestellt. Für die Suspension 2 beträgt die Intensität des Signals bei 1,22 ppm 2,48 Einheiten und die Linienbreite beträgt 0,058 ppm. Die Vergleichsformulierung V2 weist für das Signal bei 1,22 ppm eine Intensität von 5,82 Einheiten und eine Linienbreite von 0,037 ppm auf.

Der verfestigende Stoff kann in den Öltröpfchen ein Netzwerk aufbauen und so die Formstabilität bewirken. Ebenso kann der verfestigende Stoff diffus in der Matrix des flüssigen Öls verteilt sein und so die Formstabilität bewirken.

Beispiel 25:

Dieses Beispiel verdeutlicht den oben definierten Kristallinitätsindex (KI). Zunächst wird die Rohware vermessen. Die Höhe des Schmelzpeaks von z. B. Hydroxyoctacosanylhydroxystearat bei 76,5 °C beträgt 1245,9 mW pro Gramm Hydroxyoctacosanylhydroxystearat (gemessen mit einem Differenz-Wärmestromkalorimeter). Als zweites mischt man 70 Teile des Öls mittelkettige Triglyceride (Miglyol 812) mit 30 Teilen Hydroxyoctacosanylhydroxystearat, erwärmt diese Mischung auf 90 °C und läßt sie unter Rühren erkalten. Die Vermessung mit einem Differenz-Wärmestromkalorimeter ergibt nunmehr eine Höhe des Schmelzpeaks bei 68,9 °C von 310,6 mW pro Gramm Hydroxyoctacosanylhydroxystearat. Der *KI* berechnet sich somit zu:

$$KI = \frac{310,6 \ mW}{1245,9 \ mW} = 0,25$$

Die Kristallinität des Rohstoffs ist in der Mischung somit drastisch reduziert. Die Ölphase ist überwiegend nicht-kristallin.

Beispiel 26:

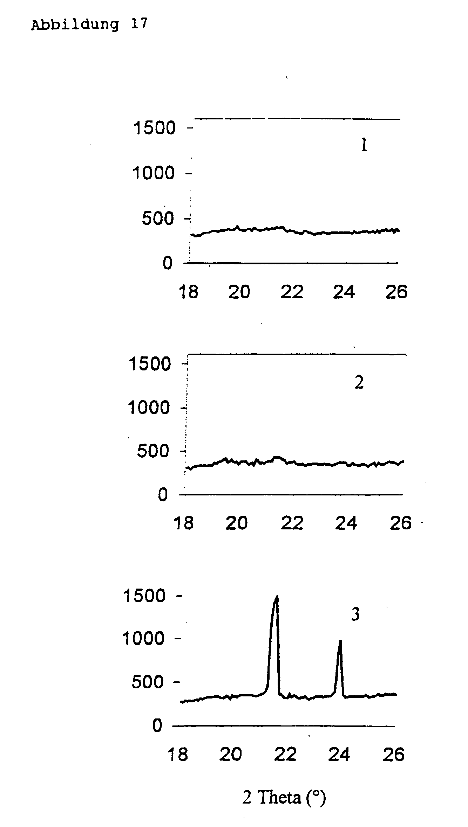

Zum Nachweis des amorphen Charakters der verfestigten Öltröpfchen kann die Röntgendiffraktometrie herangezogen werden. Abb. 17 zeigt Röntgendiffraktogramme für einen Winkelbereich von 2 Theta = 18 - 26°. In der Abb. 17 sind oben (1) das Diffraktogramm der Formulierung V2 aus Beispiel 23, in der Mitte (2) die Formulierung 2 aus Beispiel 23 und unten eine entsprechende Menge kristallines Cetylpalmitat gezeigt. Die Formulierung V2 ist, wie zu erwarten, überwiegend röntgenamorph (flüssige Öltröpfchen) (1). Die Formulierung 2 ist trotz des anwesenden Feststoffs Hydroxyoctacosanylhydroxystearat ebenso röntgenamorph und erfüllt somit die Anforderungen dieser Erfindung (2). Kristallines Cetylpalmitat zeigt hingegen zwei intensive Reflexe und ist somit nicht amorph (3). Als überwiegend amorph im Sinne dieser Erfindung kann insbesondere definiert werden, daß die Intensität (Höhe) eines Reflexes der Suspension maximal 50 % der Intensität des Reflexes des entsprechenden festen Rohmaterials, jeweils bezogen auf die gleiche Gewichtsmenge des Rohmaterials, beträgt.

Kurze Erklärung der Abbildungen:

Abb. 1:     Struktur eines Gelgerüstes aus sphärischen Aerosil (Siliciumdioxid) Partikeln.

Abb. 2:     Struktur eines polydispersen Gelgerüstes aus Magnesium-Aluminium-Silikat (Bentonit).

Abb. 3:     Bildung von Feststoffbrücken bei Kontakt von Lipidpartikeln durch Verfestigung der äußeren Schale.

Abb. 4:     Struktur einer biamphiphilen Creme.

Abb. 5:     Röntgendiffraktogramme der Imwitor-Partikeldispersion in Cremes (Beispiele 10 und 13) am Tag nach der Herstellung (links) und nach 168 Tagen Lagerung (rechts).

Abb. 6:     Kristallisationswärme (J/g) des flüssigen Lipids in den Partikeln aus Beispiel 12 als Funktion des Ölgehalts in der Lipidmischung aus flüssigem Miglyol und festem Compritol. Vergleich: Emulsion hergestellt durch Verwendung von Miglyol, d.h. Lipid besteht aus 100 % Öl. (Analyse mit DSC (Differential Scanning Calorimetry), Temperaturbereich: -20 bis -60 °C, Abkühlrate: 5 K/min, Mettler Toledo DSC 821e, Mettler, Gießen).

Abb. 7:     Röntgendiffraktogramme der Partikel mit ansteigendem Ölanteil aus Beispiel 12. Von oben nach unten: Ölanteil im Lipid: 38 %, 28 %, 16,7 % und 8,3 %.

Abb. 8:     Röntgendiffraktogramme der Imwitor-Partikeldispersion direkt nach Einarbeitung in ein Carbopol-Gel und nach Transformation in ein festes Partikel durch Elektrolytzusatz (Beispiel 13)

Abb. 9:     Röntgendiffraktogramm der Partikeldispersion aus Bei spiel 14 vor Einbringen in die Franz Zelle (Kurve unten) und nach Wasserverdunstung über der Meßzeitraum von 24 Stunden (oben).

Abb. 10:    Ansteigender Retinol Flux aus der Dispersion mit flüssig-festen Partikeln aus Beispiel 14 im Zuge des Übergangs zum festen Partikel in βi-Modifikation. Zum Vergleich: konstante Freisetzung von Retinol aus flüssigen Partikeln (Öltropfen einer Emulsion).

Abb. 11:    Röntgendiffraktogramm des flüssig-fest Lipidpartikel enthaltenden Hydrogels aus Beispiel 15 vor Einbringen in die Franz Zelle (kurve unten) und nach Wasserverdunstung über der Meßzeitraum von 24 Stunden (oben, Pfeil: Peak für βi-Modifikation).

Abb. 12:    Ansteigender Retinol Flux aus dem flüssig-fest Lipidpartikel enthaltenden Hydrogel aus Beispiel 15 im Zuge des Übergangs zum festen Partikel in βi-Modifikation. Zum Vergleich: konstante Freisetzung von Retinol aus flüssigen Partikeln (Öltropfen einer Emulsion) in identischer Hydrogelgrundlage.

Abb. 13:    Model zur Wirkstoffliberation aus Cyclosporin-Lipidpartikeln (Beispiel 6).

(fortgesetzt)

Abb. 14:  Freisetzung von Zitronenöl bei 32°C aus SLN-Dispersion und Emulsion.

Abb. 15:  Reduzierung der Reizwirkung des Wirkstoffs durch dessen Einschluß in das Trägersystem.

Abb. 16:  $^1$H-NMR Spektren der Suspension 2 (oberes Spektrum) und der Vergleichsformulierung V2 (unteres Spektrum) aus Beispiel 23.

Abb. 17:  Röntgendiffraktogramme für einen Winkelbereich von 2 Theta = 18 - 26°; oben (1) das Diffraktogramm der Formulierung V2 aus Beispiel 23, in der Mitte (2) die Formulierung 2 aus Beispiel 23 und unten eine entsprechende Menge kri-stallines Cetylpalmitat.

**Patentansprüche**

1. Lipidpartikeldispersion, **dadurch gekennzeichnet, daß** sie Lipidpartikel umfasst, die eine gemischte Matrix aus festem Lipid und flüssigem Lipid aufweisen, integer sind und eine innere Struktur aufweisen, die sich durch eine nicht voll-ständige Kristallinität in der β-Modifikation auszeichnet, wie bestimmt durch Differential Scanning Calorimetry (DSC) und Röntgendiffraktometrie, wobei das flüssige Lipid, oder eine Mischung aus flüssigen Lipiden, und das feste Lipid, oder eine Mischung aus festen Lipiden in einem Verhältnis von 50 + 50 bis 0,1 + 99,9 oder 99 + 1 bis 50 + 50 gemischt werden, die Lipidpartikel in einer äußeren Phase (Dispersionsmedium) dispergiert vorliegen (Dispersion), wobei die Lipide durch Anwendung von Hockdruckhomogenisation dispergiert werden, wobei der Zusatz an Lipidphase zu dem Dispersionsmedium in einem Schritt oder sukzessive in Teilschritten erfolgt, die Lipide liegen dabei oberhalb ihres Schmelzpunktes im flüssigen Zustand oder im festen und/oder teilweise festen Zustand vor, und die Dispersion zusammengesetzt ist aus:

   a) einem oder mehreren Ölen als Lipidpartikelbestandteil, welche bei 4 °C flüssig sind,

   b) einem oder mehreren lipophilen Stoffen als Lipidpartikelbestandteil, die bei 37 °C fest sind und das flüssige Öl verfestigen,

   c) einer Wasserphase oder mit Wasser mischbaren Phase als Dispersionsmedium,

   d) einem oder mehreren Stoffen zur Erhöhung der physikalischen Stabilität der Dispersion,

   e) keinem, einem oder mehreren Wirkstoffen, die sich überwiegend in den Lipidtröpfchen befinden.

2. Lipidpartikeldispersion nach Anspruch 1, **dadurch gekennzeichnet, daß** sie eingelagert in ihrem festen Lipidmatrixanteil flüssige oder flüssigkristalline Matrixanteile bzw. -bereiche mit einer Tröpfchen- bzw. Teilchengröße im Nanometerbereich (Nano-Compartimente) umfasst.

3. Lipidpartikeldispersion nach einem der Ansprüche 1 oder 2, **dadurch gekennzeichnet, daß** das flüssige Lipid, oder eine Mischung aus flüssigen Lipiden, und das feste Lipid, oder eine Mischung aus festen Lipiden in einem Verhältnis von 30 + 70 bis 0,1 + 99,9 gemischt werden.

4. Lipidpartikeldispersion nach einem der Ansprüche 1 bis 3, **dadurch gekennzeichnet, dass** sie ferner

   f) natürlichen Antioxidantien und Synergisten, und/oder

   g) weitere kosmetische oder pharmazeutische Wirk- und Hilfsstoffe umfasst.

5. Lipidpartikeldispersion nach Anspruch 4, **dadurch gekennzeichnet, daß** die Wirkstoffe in den wirkstoffhaltigen Lipidpartikeln lipophil sind, insbesondere ein Ciclosporin oder einen UV-Blocker umfassen, hydrophil sind, insbesondere ein Peptid oder Protein oder Hormon) oder unlöslich sind, insbesondere Titandioxid oder Magnetit umfassen.

6. Lipidpartikeldispersion nach einem der Ansprüche 1 bis 5, **dadurch gekennzeichnet, daß** die Partikel aus folgenden einzelnen Lipiden oder deren Mischungen hergestellt werden: natürliche oder synthetische Triglyceride bzw. Mischungen derselben, Monoglyceride und Diglyceride, alleine oder Mischungen derselben oder mit Triglyceriden, selbstemulgierende modifizierte Lipide, natürliche und synthetische Wachse, Fettalkohole, einschließlich ihrer Ester und Ether sowie in Form von Lipidpeptiden, Apolipoproteine oder irgendwelche Mischungen derselben.

7. Lipidpartikeldispersion nach Anspruch 6, **dadurch gekennzeichnet, daß** die Lipide synthetische Monoglyceride, Diglyceride und Triglyceride als individuelle Substanzen oder als Mischung (insbesondere Hartfett), Triglyceride (insbesondere Glyceroltrilaurat, Glycerolmyristat, Glycerolpalmitat, Glycerolstearat oder Glycerolbehenat), Wachse, insbesondere Cetylpalmitat, Carnaubawachs oder weißes Wachs (DAB) und/oder Kohlenwasserstoffe (insbesondere Hartparaffin) umfassen.

8. Lipidpartikeldispersion nach einem der Ansprüche 1 bis 7, **dadurch gekennzeichnet, dass** sie bezogen auf das Gewicht der Dispersion, einen Feststoffgehalt von 30 bis 95 % an Lipid und Stabilisator aufweist.

9. Lipidpartikeldispersion nach Anspruch 8, **dadurch gekennzeichnet, daß** die äußere Phase (das Dispersionsmedium) nichtwäßrig ist, insbesondere flüssige Polyethylenglykole (PEG) und vorzugsweise PEG 400 und/oder 600 enthält.

10. Lipidpartikeldispersion nach einem der Ansprüche 8 bis 9, **dadurch gekennzeichnet, daß** die Partikel in Dispersion durch Tenside, Stabilisatoren, insbesondere sterische Stabilisatoren und Polymere oder geladene Stabilisatoren, und/oder Antiflokkulantien einzeln oder in deren Mischung stabilisiert sind.

11. Lipidpartikeldispersion nach Anspruch 10, **dadurch gekennzeichnet, daß** die Tenside Alkaliseifen, Metallseifen, insbesondere Calciumdilaurat, Aminseifen, Alkylsulfate, Alkylsulfonate, insbesondere Mono- und Diglyceride, Fettalkohole, insbesondere Cetylalkohol und Stearylalkohol, oder Fettsäuren, Fettsäuresorbitanester, insbesondere Span, Ester und Ether von Zuckern oder von Zuckeralkoholen mit Fettsäuren oder Fettalkoholen (vorzugsweise Sucrose-Monostearat, Sucrose-Distearat, Sucrose-Cocoat, Sucrose-Stearat, Sucrose-Dipalmitat, Sucrose-Palmitat, Sucrose-Laurat, Sucrose-Octanoat, Sucrose-Oleat), und/oder natürliche Tenside, insbesondere Saponine, umfassen.

12. Lipidpartikeldispersion nach Anspruch 10 oder 11, **dadurch gekennzeichnet, daß** die geladenen ionischen Stabilisatoren Diacetylphosphate, Phosphatidylglycerin, Lecithine unterschiedlicher Herkunft (insbesondere Eilecithin oder Sojalecithin), chemisch modifizierte Lecithine (insbesondere hydrierte Lecithine), Phospholipide und Sphingolipide, Mischung von Lecithinen mit Phospholipiden, Sterole (insbesondere Cholesterol, Cholesterol-Derivate oder Stigmasterin) und/oder gesättigte und ungesättigte Fettsäuren, gallensaure Salze, Natriumcholat, Natriumglycocholat, Natriumtaurocholat, Natriumdeoxycholat oder ihre Mischungen, Aminosäuren und quartäre Ammoniumverbindungen umfassen.

13. Lipidpartikeldispersion nach einem der Ansprüche 10 bis 12, **dadurch gekennzeichnet, daß** die Anti-Flokkulantien Natriumcitrat, Natriumpyrophosphat und/oder Natriumsorbat umfassen.

14. Lipidpartikeldispersion nach einem der Ansprüche 10 bis 13, **dadurch gekennzeichnet, daß** viskositätserhöhende Substanzen, insbesondere Cellulose-Ether oder Cellulose-Ester, (vorzugsweise Methylcellulose, Hydroxyethylcellulose, Hydroxypropylcellulose, Natriumcarboxymethylcellulose), Polyvinylderivate, insbesondere Polyvinylalkohol, Polyvinylpyrrolidon, Polyvinylacetat, Alginate, Polyacrylate (vorzugsweise Carbopol), Xanthane und/oder Pektine zugesetzt sind.

15. Lipidpartikeldispersion nach einem der Ansprüche 10 bis 14, **dadurch gekennzeichnet, daß** die Stabilisatoren und Antiflokkulantien in einer Konzentration von 0,001 % bis 30 %, vorzugsweise mit 0,01 % bis 20 % (m/m) und insbesondere in einer Menge von 0,05 % bis zu 10 % einzeln oder in deren Mischung in der Dispersion enthalten sind, die viskositätserhöhende Substanzen sind, wenn vorhanden, in einer Konzentration von 0,001 % bis 30 %, vorzugsweise in einer Menge von 0,01 bis 20 % und insbesondere in einer Menge von 0,1 % bis 10 % (m/m) und vorzugsweise im Bereich zwischen 0,5 % und 5 % einzeln oder in deren Mischung enthalten sind.

16. Lipidpartikeldispersion nach einem der Ansprüche 8 bis 15, **dadurch gekennzeichnet, daß** die mit Laserdiffraktometrie bestimmte mittlere Partikelgröße (Durchmesser 50%, Anzahlverteilung) im Bereich 0,03 $\mu$m bis 50 $\mu$m liegt.

17. Lipidpartikeldispersion nach Anspruch 16, **dadurch gekennzeichnet, daß** die mittlere Partikelgröße im Bereich 0,03 $\mu$m bis 10 $\mu$m liegt.

18. Lipidpartikeldispersion nach Anspruch 13, **dadurch gekennzeichnet, daß** die mittlere Partikelgröße im Bereich 0,03 $\mu$m bis 1 $\mu$m liegt.

19. Lipidpartikeldispersion nach einem der Ansprüche 8 bis 18 **dadurch gekennzeichnet, daß** sie aseptisch hergestellt worden ist, sterilisiert worden ist und/oder parenteral applizierbar ist.

20. Lipidpartikeldispersion nach einem der Ansprüche 1 bis 18 beladen mit natürlichen, halbsynthetischen und synthetischen Cyclosporinen, insbesondere zur Anwendung auf der Haut und im Gastrointestinaltrakt.

21. Lipidpartikeldispersion nach einem der Ansprüche 1 bis 18 **dadurch gekennzeichnet, daß** sie natürliche, synthetische, halbsynthetische Duftstoffe einzeln oder in Mischung enthält, insbesondere ätherische Öle, deren isolierte Duftstoffe, Parfüms, Pheromone oder Repellents.

22. Lipidpartikeldispersion nach Anspruch 21, **dadurch gekennzeichnet, daß** sie als ätherische Öle Zitronenöl, Rosenöl, Lavendelöl, Bergamottöl, Melissenöl, Nelkenöl, Zimtöl, Orangenöl, Jasminöl, Rosmarinöl, Anisöl, Pfefferminzöl, Sandelholzöl Ylang-Ylang-Öl oder deren isolierte Inhaltsstoffe, insbesondere 1,8-Cineol, Menthol, Terpinhydrat, Limonen, $\alpha$-Pinen oder Eugenol, enthält.

23. Lipidpartikeldispersion nach Anspruch 21, **dadurch gekennzeichnet, daß** sie natürliche Repellents, insbesondere Citrusöle, Eukalyptusöl oder Campher, oder synthetische Repellents, insbesondere N,N-Diethyltoluamid (DEET), Dibutylphthalat, Dimethylphthalat oder 2-Ethyl-1,3-hexandiol enthält.

24. Lipidpartikeldispersion nach einem der Ansprüche 1 bis 18, **dadurch gekennzeichnet, daß** sie Marker enthält, insbesondere radioaktive Verbindungen, Farbstoffe und fluoreszierende Farbstoffe, Eisenoxide wie Magnetit, insbesondere als kleine Eisenoxidpartikel im Bereich ca. 1 bis 3 nm, einzeln oder in Mischungen.

25. Lipidpartikeldispersion nach Anspruch 24, **dadurch gekennzeichnet, daß** sie als radioaktive Verbindungen Jod-Isotope, Technetium-Isotope, Indium-Isotope als Ionen oder als Bestandteil von Molekülen enthält.

26. Lipidpartikeldispersion nach Anspruch 24, **dadurch gekennzeichnet, daß** sie als Farbstoff Sudanrot und als Fluoreszenzfarbstoffe Nile Red und Fluorescein enthält.

27. Lipidpartikeldispersion nach einem der Ansprüche 1 bis 18, **dadurch gekennzeichnet, daß** sie Lipide mit ausreichender Veränderung der T1 und T2-Relaxationszeit enthält, so daß sie als Kontrastmittel in der Magnetresonanztomographie eingesetzt werden kann.

28. Lipidpartikeldispersion nach einem der Ansprüche 1 bis 18, **dadurch gekennzeichnet, daß** sie als Wirkstoffe Gifte enthält.

29. Lipidpartikeldispersion nach Anspruch 28, **dadurch gekennzeichnet, daß** sie als Gifte chlorierte Kohlenwasserstoffe, insbesondere $\gamma$-Hexachlorcyclohexan, Pyrethrine, Pyrethroide, Alkylphosphate, insbesondere Paraoxon, Parathion, Fenthion, Dichlorvos und Carbaminsäureester, insbesondere Butoxicarboxim, Bendiocarb, Methomyl oder Proxopur enthält.

30. Lipidpartikeldispersion nach einem der vorhergehenden Ansprüche, **dadurch gekennzeichnet, daß** das flüssige Öl eine Verbindung eines kurzkettigen (14 oder weniger Kohlenstoffatome) Fettalkohols ist.

31. Lipidpartikeldispersion nach einem der vorhergehenden Ansprüche, **dadurch gekennzeichnet, daß** das Öl aus der Gruppe Isopropylmyristat, -palmitat, -stearat, Octyldodecanol, $C_{6-14}$-Dicarbonsäurediester des Isopropylalkohols, $C_{14-20}$ verzweigtkettige, aliphatische Fettalkohole, $C_{6-19}$-Fettsäuretri-glyceride und -diglyceride, $C_{12-16}$-Oktanoate und Tridecylsalicylate gewählt wird.

32. Lipidpartikeldispersion nach einem der vorhergehenden Ansprüche, **dadurch gekennzeichnet, daß** der Stoff zur Verfestigung des flüssigen Öls ein Lipid mit einem Schmelzpunkt über 40 °C ist und insbesondere eine Verbindung eines langkettigen (18 oder mehr Kohlenstoffatome) Fettalkohols ist.

33. Lipidpartikeldispersion nach einem der vorhergehenden Ansprüche, **dadurch gekennzeichnet, daß** der verfestigende Stoff aus der Gruppe Carnaubawachs, Hydroxyoctacosanylhydroxystearat, chinesischer Wachs, Cetylpalmitat, Bienenwachs oder ähnlicher Wachse gewählt wird.

34. Lipidpartikeldispersion nach einem der vorhergehenden Ansprüche, **dadurch gekennzeichnet, daß** das flüssige Öl, oder Mischungen daraus, und der verfestigende Stoff, oder Mischungen daraus, in einem Verhältnis von 95 + 5 bis 80 + 20, gemischt werden.

35. Lipidpartikeldispersion nach einem der vorhergehenden Ansprüche, **dadurch gekennzeichnet, daß** die wäßrige Phase (Dispersionsmittel) einen Gelbildner zur Verdickung enthält, insbesondere daß die wäßrige Phase durch Verwendung eines hydrophilen Gelbildners halbfest ist, die Fließgrenze über 5 Pa bei 21 °C liegt und dieser Gelbildner aus der Gruppe Alginate, Zellulosederivate, Xanthan Gummi, Stärke, Bentonite und Glycerolmonostearat, insbesondere aus der Gruppe von polyelektrolytischen Polymeren, bevorzugt Polyacrylsäure, Carboxymethylcellulose oder Carrageenan, gewählt wird.

36. Verwendung von Lipidpartikeldispersionen gemäß einem der Ansprüche 8 bis 18 als Granulierflüssigkeit in einem Granulierungsprozeß zur Herstellung eines trockenen Granulats (insbesondere zur Abfüllung als Sachets oder in Kapseln) oder nach Komprimierung eines solchen Granulats zur Herstellung einer Tablette.

37. Verwendung von Lipidpartikeldispersionen gemäß einem der Ansprüche 8 bis 18 als Anteigflüssigkeit für eine Extrusionsmasse zur Herstellung eines trockenen Produkts, insbesondere von Pellets.

38. Verwendung von Lipidpartikeldispersionen gemäß einem der Ansprüche 8 bis 18, **dadurch gekennzeichnet, daß** die Dispersionen bei Verwendung einer nicht-wäßrigen äußeren Phase in Weichgelatinekapseln abgefüllt wird.

39. Verwendung von Lipidpartikeldispersionen gemäß einem der Ansprüche 8 bis 18 als Salbe (insbesondere streichfähige Salbe) oder Lotion (insbesondere viskose Lotion) zur topischen Applikation, wobei die Dispersion eine ausreichend hohe bis mittlere Konsistenz aufweist, die gegebenenfalls durch Zusatz eines Gelbildners zur äußeren Phase oder zusätzlicher lipophiler Phase (insbesondere Öl in dispergierter Form) noch erhöht wird.

40. Verwendung von Lipidpartikeldispersion gemäß einem der vorhergehenden Ansprüche zur Stabilisierung und/oder verlangsamten Freisetzung und/oder zur Verminderung der Reizwirkung von überwiegend in den Öltröpfchen eingeschlossenen Wirkstoffen, insbesondere bei oraler, parenteraler oder topischer Anwendung.

**Claims**

1. Lipid particle dispersion, **characterized in that** it comprises lipid particles that have a mixed matrix of solid and liquid lipids, are integer and have an inner structure which is **characterized by** an incomplete crystallinity in the β-modification, as determined by differential scanning calorimetry (DSC) and x-ray diffractometry, wherein the liquid lipid, or a mixture of liquid lipids, and the solid lipid, or a mixture of solid lipids, are mixed in a ratio of 50 + 50 to 0.1 + 99.9 or 99 + 1 to 50 + 50, the lipid particles are dispersed (dispersion) in an outer phase (dispersion media), wherein the lipids are dispersed using high pressure homogenisation, wherein the addition of lipid phase to the dispersion medium takes place in one step or successively in sub-steps, wherein the lipids are present above their melting point in liquid state or in solid and/or partly solid state and the dispersion is composed of:

    a) one or more oils as lipid particle ingredient, which are liquid at 4°C,
    b) one or more lipophilic compounds as lipid particle ingredient, which are solid at 37 °C and solidify the liquid oil,
    c) a water phase or a water miscible phase as dispersion media,
    d) one or more compounds for enhancing the physical stability of the dispersion,
    e) no, one or more active agents, which are present predominantly in the lipid droplets.

2. Lipid particle dispersion as in claim 1, **characterized in that** comprises enclosed in its solid lipid matrix portion liquid or liquid crystalline matrix fractions or regions with a droplet or particle size in the nanometer range (nano compartiments).

3. Lipid particle dispersion as in any of claims 1 or 2, **characterized in that** the liquid lipid, or a mixture of liquid lipids, is mixed with the solid lipid, or a mixture of solid lipids, in a ratio of 30 + 70 to 0.1 + 99.9.

4. Lipid particle dispersion as in any of claims 1 to 3, **characterized in that** it further comprises

    f) natural antioxidants and synergists, and/or
    g) further cosmetically or pharmaceutically active agents and excipients.

5. Lipid particle dispersion as in claim 4, **characterized in that** the active agents within the lipid particles containing the active agents are lipophilic, in particular comprise cyclosporin or a UV-blocker, are hydrophilic, in particular comprise a peptide or protein or hormone, or are insoluble, in particular comprise titanium dioxide or magnetite.

6. Lipid particle dispersion as in any of claims 1 to 5, **characterized in that** the particles are produced from the following single lipids or their mixtures: natural or synthetic triglycerides or mixtures thereof, monoglycerides and diglycerides, alone or mixtures thereof or with triglycerides, self-emulgating modified lipids, natural or synthetic waxes, fatty alcohols including their esters and ethers as well as in form of lipid peptides, apolipoproteins or any mixtures thereof.

7. Lipid particle dispersion as in claim 6, **characterized in that** the lipids comprise synthetic monoglycerides, diglycerides and triglycerides as individual substances or in mixtures (in particular hydrogenated fat), triglycerides (in particular glyceroltrilaurate, glycerolmyristate, glycerolpalminate, glycerolstearate or glycerolbehenate), waxes, in particular cetylpalminate, carnauba wax or white wax (DAB) and/or hydrocarbons (in particular hard paraffin).

8. Lipid particle dispersion as in any of claims 1 to 7, **characterized in that** it has a solid content of 30 to 95% of lipids and stabilizer based on the weight of the dispersion.

9. Lipid particle dispersion as in claim 8, **characterized in that** the outer phase (the dispersion media) is non-aqueous, in particular comprises liquid polyethylene glycols (PEG), preferably PEG 400 and/or PEG 600.

10. Lipid particle dispersion as in any of claims 8 to 9, **characterized in that** the particles in dispersion are stabilised by surfactants, stabilising agents, in particular steric stabilising agents and polymers or charged stabilising agents, and/or anti-flocculants alone or in mixtures thereof.

11. Lipid particle dispersion as in claim 10, **characterized in that** surfactants comprise alkali soaps, metal soaps, in particular calcium dilaurate, amine soaps, alkyl sulfates, alkyl sulfonates, in particular mono- and diglyerides, fatty alcohols, in particular cetylalcohol and stearylalcohol, or fatty acids, fatty acid sorbitan esters, in particular Span, esters and ethers of sugars or of sugar alcohols with fatty acids or fatty acid alcohols (in particular sucrose monostearate, sucrose distearate, sucrose cocoate, sucrose stearate, sucrose dipalminate, sucrose palminate, sucrose laurate, sucrose octanoate, sucrose oleate), and/or natural surfactants, in particular saponines.

12. Lipid particle dispersion as in claim 10 or 11, **characterized in that** the charged ionic stabilising agents comprise diacetyl phosphate, phosphate diglycerol, lecitins of different origin (in particular egg lecitin or soy lecitin), chemically modified lecitins (in particular hydrogenated lecitins), phospholipids and sphingolipids, mixtures of lecitins with phospholipids, sterols (in particular cholesterol, cholesterol derivatives or stigmasterol) and/or saturated and unsaturated fatty acids, bile acid salts, sodium cholate, sodium glycocholate, sodium taurocholate, sodium deoxycholate or their mixtures, amino acids and quarternary ammonia compounds.

13. Lipid particle dispersion as in any of claims 10 to 12, **characterized in that** the anti-flocculants comprise sodium citrate, sodium pyrophosphate and/or sodium sorbate.

14. Lipid particle dispersion as in any of claims 10 to 13, **characterized in that** viscosity-enhancing compounds, in particular cellulose ethers or cellulose esters (preferably methyl cellulose, hydroxyethyl cellulose, hydroxypropyl cellulose, sodium carboxymethyl cellulose), polyvinyl derivatives, in particular polyvinyl alcohol, polyvinyl pyrrolidone, polyvinyl acetate, alginates, polyacrylates (preferably Carbopol), xantanes and/or pektins are added.

15. Lipid particle dispersion as in any of claims 10 to 14, **characterized in that** the stabilizing agents and antiflocculants are present in the dispersion in a concentration of 0.001 to 30%, preferably 0.01 to 20% (wt/wt) and in particular in an amount of 0.05 up to 10%, individually or in mixtures thereof, the viscosity-enhancing compounds are, if present, present in an amount of 0.001 to 30%, preferably in an amount of 0.01 to 20% and in particular in an amount of 0.1 to 10% (wt/wt) and preferably in a range between 0.5 and 5%, individually or in mixtures thereof.

16. Lipid particle dispersion as in any of claims 8 to 15, **characterized in that** the average particle size determined by laser-diffractometry (diameter 50%, number distribution) is in the range of 0.03 to 50 $\mu$m.

17. Lipid particle dispersion as in claim 16, **characterized in that** the average particle size is in the range of 0.03 to 10 $\mu$m.

18. Lipid particle dispersion as in claim 13, **characterized in that** the average particle size is in the range of 0.03 to 1 $\mu$m.

19. Lipid particle dispersion as in any of claims 8 to 18, **characterized in that** it has been aseptically produced, has been sterilized and/or is parenterally applicable.

**20.** Lipid particle dispersion as in any of claims 1 to 18 loaded with natural, semi-synthetic and synthetic cyclosporins, in particular for applications on the skin and in the gastrointestinal tract.

**21.** Lipid particle dispersion as in any of claims 1 to 18, **characterized in that** it contains natural, synthetic, semi-synthetic fragrances individually and in mixtures, in particular essential oils, their isolated fragrances, parfumes, pheromones or repellents.

**22.** Lipid particle dispersion as in claim 21, **characterized in that** it comprises as essential oils citric oil, rose oil, lavender oil, bergamot oil, melissa oil, clove oil, cinnamon oil, orange oil, jasmine oil, rosemary oil, anise oil, peppermint oil, sandalwood oil, ylang-ylang oil or their isolated ingredients, in particular 1,8-cineol, menthol, terpin hydrate, limonene, $\alpha$-pinen or eugenol.

**23.** Lipid particle dispersion as in claim 21, **characterized in that** it comprises natural repellents, in particular citric oils, eucalyptus oil or campher, or synthetic repellents, in particular N,N-diethyltoluamide (DEET), dibutyl-phthalate, dimethylphthalate or 2-ethyl-1,3-hexanediol.

**24.** Lipid particle dispersion as in any of claims 1 to 18, **characterized in that** it comprises markers, in particular radioactive compounds, dyes, and fluorescent dyes, iron oxides like magnetite, in particular in form of small iron oxide particles in the range of 1 to 3 nm, individually or in mixtures.

**25.** Lipid particle dispersion as in claim 24, **characterized in that** it contains as radioactive compounds iodine isotopes, technetium isotopes, indium isotopes, in form of ions or in form of compounds of molecules.

**26.** Lipid particle dispersion as in claim 24, **characterized in that** it contains as dye Sudan red and as fluorescent dyes Nile red and fluorescein.

**27.** Lipid particle dispersion as in any of claims 1 to 18, **characterized in that** it comprises lipids with a sufficient shift of T1 and T2 relaxation times, so that it can be applied as contrast agent in magnetic resonance tomography.

**28.** Lipid particle dispersion as in any of claims 1 to 18 comprising poisons as active agents.

**29.** Lipid particle dispersion as in claim 28, **characterized in that** it contains as poisons chlorinated hydrocarbons, in particular $\gamma$-hexachloro cyclohexane, pyrethrins, pyrethroids, alkyl phosphates, in particular Paraoxon, Parathion, Fenthion, Dichlorvos and carbamic acid esters, in particular butoxicaboxim, bendiocarb, methomyl or proxopur.

**30.** Lipid particle dispersion as any of the previous claims, **characterized in that** the liquid oil is a compound of a short-chain (14 or less carbon atoms) fatty alcohol.

**31.** Lipid particle dispersion as in any of the previous claims, **characterized in that** the oil is selected from the group consisting of isopropyl myristate, isopropyl palmitate, isopropyl stearate, octyl dodecanole, $C_6$-$C_{14}$ dicarboxylic acid diester of isopropyl alcohol, $C_{14}$-$C_{20}$ branched-chain aliphatic fatty alcohols, $C_{6-14}$-fatty acid triglycerids and -diglycerids, $C_{12-16}$-octanoates and tridecyl salicylates.

**32.** Lipid particle dispersion in as any of the previous claims, **characterized in that** the compound for solidifaction of the liquid oil is a lipid having a melting point above 40 °C and in particular a compound of a long-chain (18 or more carbon atoms) fatty alcohol.

**33.** Lipid particle dispersion as in any of the previous claims, **characterized in that** the solidifying compound is selected from the group consisting of carnauba wax, hydroxy octacosanyl hydroxystearate, China wax, cetylpalmitate, bees wax or similar waxes.

**34.** Lipid particle dispersion as in any of the previous claims, **characterized in that** the liquid oil or mixtures thereof and the solidifying compound or mixtures thereof are mixed in a ratio of 95 + 5 to 80 + 20.

**35.** Lipid particle dispersion as in any of the previous claims, **characterized in that** the aqueous phase (dispersant) contains a gelling agent for thickening, in particular that, due to the use of a hydrophilic gelling agent, the aqueous phase is semi-solid, the flow limit is above 5 Pa at 21 °C and the gelling agent is selected from the group consisting of alginates, cellulose derivatives, xanthan gums, starch, bentonites and glycerol monostearate, in particular from

26

the group consisting of polyelectrolytic polymers, preferred polyacrylic acid, carboxymethylic cellulose or Carrageenan.

36. Use of lipid particle dispersions according to one of claims 8 to 18 as granulating liquid in a granulation process for the production of a dry granulate (in particular for the filling as sachet or in capsules) or after compression of such granulate for the production of a tablet.

37. Use of lipid particle dispersions according to one of claims 8 to 18 as paste-forming-liquid for an extrusion mass for the production of a dry product, in particular pellets.

38. Use of lipid particle dispersions according to one of claims 8 to 18, **characterized in that**, by using a non-aqueous outer phase, the dispersion is filled into soft-gelatine capsules.

39. Use of lipid particle dispersions according to one of claims 8 to 18 as ointment (in particular an easy to spread ointment) or lotion (in particular a viscous lotion) for topical application, whereby the dispersion has a sufficient high to medium consistency, that, optionally is further enhanced by adding a gelling agent to the outer phase or additional lipophilic phase (in particular oil in dispersed form).

40. Use of lipid particle dispersion according to any previous claim for the stabilisation and/or retarded release and/or decrease of the irritating effect of active agents predominantly encapsulated within the oil droplets, in particular for oral, parenteral or topical application.


**Revendications**

1. Dispersion de particules lipidiques, **caractérisée en ce qu'**elle comprend des particules lipidiques qui comprennent une matrice mixte constituée d'un lipide solide et d'un lipide liquide, sont intègres et présentent une structure interne, qui se **caractérise par** une cristallinité incomplète dans la modification β, telle que déterminée par analyse calorimétrique différentielle (ACD) et diffractométrie aux rayons X, le lipide liquide, ou un mélange de lipides liquides, et le lipide solide, ou un mélange de lipides solides, étant mélangés selon un rapport de 50 + 50 à 0,1 + 99,9 ou de 99 + 1 à 50 + 50, les particules lipidiques étant présentes en dispersion dans une phase extérieure (milieu de dispersion) (dispersion), les lipides étant dispersés par utilisation d'une homogénéisation sous haute pression, l'addition de la phase lipidique au milieu de dispersion ayant lieu dans le cadre d'une étape ou successivement dans le cadre d'étapes partielles, les lipides se trouvant alors au-delà de leur point de fusion à l'état liquide, et/ou à l'état solide et/ou partiellement solide, et la dispersion étant composée :

a) d'une ou plusieurs huiles, en tant que constituants de particules lipidiques, qui sont liquides à 4°C,
b) d'une ou plusieurs substances lipophiles, en tant que constituants des particules lipidiques, qui sont solides à 37°C et solidifient l'huile liquide,
c) d'une phase aqueuse ou d'une phase miscible à l'eau, en tant que milieu de dispersion,
d) d'une ou plusieurs substances pour augmenter la stabilité physique de la dispersion,
e) d'aucune, d'une ou de plusieurs substances actives, qui pour l'essentiel se trouvent dans les gouttelettes lipidiques.

2. Dispersion de particules lipidiques selon la revendication 1, **caractérisée en ce qu'**elle comprend, incorporés dans sa fraction de matrice lipidique solide, des fractions ou domaines de matrices liquides ou cristallins liquides, ayant une grosseur de gouttelettes ou une grosseur de grain dans le domaine nanométrique (nano-compartiments).

3. Dispersion de particules lipidiques selon l'une des revendications 1 ou 2, **caractérisée en ce que** le lipide liquide, ou un mélange de lipides liquides, et le lipide solide, ou un mélange de lipides solides, sont mélangés selon un rapport de 30 + 70 à 0,1 + 99,9.

4. Dispersion de particules lipidiques selon l'une des revendications 1 à 3, **caractérisée en ce qu'**elle comprend en outre

f) des antioxydants et synergistes naturels, et/ou
g) des substances actives ou adjuvants cosmétiques ou pharmaceutiques supplémentaires.

**5.** Dispersion de particules lipidiques selon la revendication 4, **caractérisée en ce que** les substances actives sont lipophiles dans les particules lipidiques contenant des substances actives, comprennent en particulier une cyclosporine ou un agent bloquant les UV, sont hydrophiles, en particulier un peptide ou une protéine ou une hormone, ou sont insolubles, en particulier du dioxyde de titane ou de la magnétite.

**6.** Dispersion de particules lipidiques selon l'une des revendications 1 à 5, **caractérisée en ce que** les particules sont fabriquées à partir des lipides individuels suivants, ou de leurs mélanges : des triglycérides naturels ou synthétiques, ou des mélanges de ceux-ci, des monoglycérides et des diglycérides, à titre individuel ou sous forme de mélanges entre eux ou avec des triglycérides, des lipides modifiés auto-émulsifiants, des cires naturelles et synthétiques, des alcools gras, y compris leurs esters et éthers, ainsi que sous forme de peptides lipidiques, d'apolipoprotéines ou de quelques mélanges que ce soit de ceux-ci.

**7.** Dispersion de particules lipidiques selon la revendication 6, **caractérisée en ce que** les lipides comprennent des monoglycérides, des diglycérides et des triglycérides synthétiques, sous forme de substances individuelles ou d'un mélange (en particulier une graisse dure), des triglycérides (en particulier le trilaurate de glycérol, le myristate de glycérol, le palmitate de glycérol, le stéarate de glycérol ou le béhénate de glycérol), des cires, en particulier le palmitate de cétyle, la cire de carnauba ou la cire blanche (DAB) et/ou des hydrocarbures (en particulier la paraffine dure).

**8.** Dispersion de particules lipidiques selon l'une des revendications 1 à 7, **caractérisée en ce qu'**elle présente, par rapport au poids de la dispersion, une teneur en extrait sec de 30 à 95 % en lipides et stabilisants.

**9.** Dispersion de particules lipidiques selon la revendication 8, **caractérisée en ce que** la phase extérieure (le milieu de dispersion) est non aqueuse, et contient en particulier des polyéthylèneglycoles (PEG) liquides, et de préférence du PEG 400 et/ou 600.

**10.** Dispersion de particules lipidiques selon l'une des revendications 8 à 9, **caractérisée en ce que** les particules sont, dans la dispersion, stabilisées par des tensioactifs, des stabilisants, en particulier des stabilisants stériques et des polymères ou des stabilisants chargés, et/ou des anti-floculants, à titre individuel ou en mélange.

**11.** Dispersion de particules lipidiques selon la revendication 10, **caractérisée en ce que** les tensioactifs comprennent des savons alcalins, des savons métalliques, en particulier du dilaurate de calcium, des savons d'amines, des alkylsulfates, des alkylsulfonates, en particulier des mono-et des diglycérides, des alcools gras, en particulier l'alcool cétylique et l'alcool stéarylique, ou des acides gras, des esters d'acides gras du sorbitan, en particulier le Span, des esters et éthers de sucres ou d'alcools de sucre et d'acides gras ou d'alcools gras (de préférence le monostéarate de saccharose, le distéarate de saccharose, le cocoate de saccharose, le stéarate de saccharose, le dipalmitate de saccharose, le palmitate de saccharose, le laurate de saccharose, l'octanoate de saccharose, l'oléate de saccharose), et/ou des tensioactifs naturels, en particulier les saponines.

**12.** Dispersion de particules lipidiques selon la revendication 10 ou 11, **caractérisée en ce que** les stabilisants ioniques chargés comprennent des diacétylphosphates, du phosphatidylglycérol, des lécithines de différentes origines (en particulier la lécithine du jaune d'oeuf ou la lécithine du soja), des lécithines chimiquement modifiées (en particulier les lécithines hydrogénées), des phospholipides et des sphingolipides, un mélange de lécithines et de phospholipides, des stérols (en particulier le cholestérol, les dérivés du cholestérol ou la stigmastérine) et/ou des acides gras saturés ou insaturés, des sels de l'acide gallique, du cholate de sodium, du glycocholate de sodium, du taurocholate de sodium, du désoxycholate de sodium ou des mélanges de ceux-ci, des acides aminés et des composés de l'ammonium quaternaire.

**13.** Dispersion de particules lipidiques selon l'une des revendications 10 à 12, **caractérisée en ce que** les anti-floculants comprennent le citrate de sodium, le pyrophosphate de sodium et/ou le sorbate de sodium.

**14.** Dispersion de particules lipidiques selon l'une des revendications 10 à 13, **caractérisée en ce que** des substances augmentant la viscosité sont ajoutées, en particulier des éthers de cellulose ou des esters de cellulose (de préférence la méthylcellulose, l'hydroxyéthylcellulose, l'hydroxypropylcellulose, la carboxyméthylcellulose sodique), des dérivés polyvinyliques, en particulier le poly(alcool vinylique), de la polyvinylpyrrolidone, du poly(acétate de vinyle), des alginates, des polyacrylates (de préférence du Carbopol), des xanthanes et/ou des pectines.

**15.** Dispersion de particules lipidiques selon l'une des revendications 10 à 14, **caractérisée en ce que** les stabilisants et anti-floculants sont contenus dans la dispersion selon une concentration de 0,001 % à 30 %, de préférence de 0,01 % à 20 % (m/m), et en particulier en une quantité de 0,05 % à 10 %, à titre individuel ou en mélange, les substances augmentant la viscosité étant, quand elles sont présentes, contenues selon une concentration de 0,001 % à 30 %, de préférence en une quantité de 0,01 à 20 % et en particulier en une quantité de 0,1 % à 10 % (m/m) et de préférence en une quantité comprise dans la plage de 0,5 % à 5 %, à titre individuel ou en mélange.

16. Dispersion de particules lipidiques selon l'une des revendications 8 à 15, **caractérisée en ce que** la grosseur de grain moyenne déterminée par diffractométrie laser (diamètre 50 %, répartition en nombre) est comprise dans la plage de 0,03 $\mu$m à 50 $\mu$m.

17. Dispersion de particules lipidiques selon la revendication 16, **caractérisée en ce que** la grosseur de grain moyenne est comprise dans la plage de 0,03 $\mu$m à 10 $\mu$m.

18. Dispersion de particules lipidiques selon la revendication 13, **caractérisée en ce que** la grosseur de grain moyenne est comprise dans la plage de 0,03 $\mu$m à 1 $\mu$m.

19. Dispersion de particules lipidiques selon l'une des revendications 8 à 18, **caractérisée en ce qu'**elle a été fabriquée dans des conditions aseptiques, a été stérilisée et/ou peut être administrée par voie parentérale.

20. Dispersion de particules lipidiques selon l'une des revendications 1 à 18, chargée de cyclosporines naturelles, semi-synthétiques et synthétiques, en particulier pour utilisation sur la peau et dans le tractus gastro-intestinal.

21. Dispersion de particules lipidiques selon l'une des revendications 1 à 18, **caractérisée en ce qu'**elle contient des substances aromatiques naturelles, synthétiques, semi-synthétiques, à titre individuel ou en mélange, en particulier des huiles essentielles, des substances aromatiques, parfums, phéromones ou répulsifs isolés de ces dernières.

22. Dispersion de particules lipidiques selon la revendication 21, **caractérisée en ce qu'**elle contient en tant qu'huiles essentielles de l'essence de citron, de l'essence de rose, de l'essence de lavande, de l'essence de bergamote, de l'essence de mélisse, de l'essence de girofle, de l'essence de cannelle, de l'essence d'orange, de l'essence de jasmin, de l'essence de romarin, de l'essence d'anis, de l'essence de menthe poivrée, de l'essence de bois de santal, de l'essence d'ylang-ylang ou leurs constituants isolés, en particulier le 1,8-cinéol, le menthol, l'hydrate de terpine, le limonène, l'$\alpha$-pinène ou l'eugénol.

23. Dispersion de particules lipidiques selon la revendication 21, **caractérisée en ce qu'**elle contient des répulsifs naturels, en particulier des essences d'agrumes, de l'essence d'eucalyptus ou du camphre, ou des répulsifs synthétiques, en particulier le N,N-diéthyltoluamide (DEET), le phtalate de dibutyle, le phtalate de diméthyle ou le 2-éthyl-1,3-hexanediol.

24. Dispersion de particules lipidiques selon l'une des revendications 1 à 18, **caractérisée en ce qu'**elle contient des marqueurs, en particulier des composés radioactifs, des colorants et des colorants fluorescents, des oxydes de fer tels que la magnétite, en particulier sous forme de petites particules d'oxyde de fer ayant une grosseur de grain comprise dans la plage d'environ 1 à 3 nm, à titre individuel ou en mélange.

25. Dispersion de particules lipidiques selon la revendication 24, **caractérisée en ce qu'**elle contient en tant que composés radioactifs des isotopes de l'iode, des isotopes du technétium, des isotopes de l'indium, sous forme d'ions ou sous forme d'un constituant de molécules.

26. Dispersion de particules lipidiques selon la revendication 24, **caractérisée en ce qu'**elle contient en tant que colorant du rouge Soudan et en tant que colorants fluorescents du rouge du Nil ou de la fluorescéine.

27. Dispersion de particules lipidiques selon l'une des revendications 1 à 18, **caractérisée en ce qu'**elle contient des lipides présentant une variation suffisante du temps de relaxation T1 et T2, de façon qu'on puisse les utiliser en tant que produits de contraste en tomographie par résonance magnétique.

28. Dispersion de particules lipidiques selon l'une des revendications 1 à 18, **caractérisée en ce qu'**elle contient en tant que substances actives des poisons.

29. Dispersion de particules lipidiques selon la revendication 28, **caractérisée en ce qu'**elle contient en tant que poisons des hydrocarbures chlorés, en particulier du γ-hexachlorocyclohexane, des pyréthrines, des pyréthroïdes, des alkylphosphates, en particulier le paraoxon, le parathion, le fenthion, le dichlorvos et les esters de l'acide carbamique, en particulier le butoxycarboxime, le bendiocarb, le méthomyle ou le proxopur.

30. Dispersion de particules lipidiques selon l'une des revendications précédentes, **caractérisée en ce que** l'huile liquide est un composé d'un alcool gras à courte chaîne (14 atomes de carbone, ou moins).

31. Dispersion de particules lipidiques selon l'une des revendications précédentes, **caractérisée en ce que** l'huile est choisie dans le groupe comprenant le myristate, le palmitate, le stéarate d'isopropyle, l'octyldodécanol, les diesters d'un acide dicarboxylique en $C_{6-14}$ de l'alcool isopropylique, les alcools gras aliphatiques à chaîne ramifiée en $C_{14-20}$, les triglycérides et diglycérides d'acides gras en $C_{6-14}$, les octanoates en $C_{12-16}$ et les tridécylsalicylates.

32. Dispersion de particules lipidiques selon l'une des revendications précédentes, **caractérisée en ce que** la substance destinée à solidifier l'huile liquide est un lipide ayant un point de fusion supérieur à 40°C, et en particulier un composé d'un alcool gras à longue chaîne (18 atomes de carbone, ou plus).

33. Dispersion de particules lipidiques selon l'une des revendications précédentes, **caractérisée en ce que** la substance solidifiante est choisie dans le groupe comprenant la cire de carnauba, l'hydroxystéarate d'hydroxyoctacosanyle, la cire de Chine, le palmitate de cétyle, la cire d'abeille ou des cires analogues.

34. Dispersion de particules lipidiques selon l'une des revendications précédentes, **caractérisée en ce que** l'huile liquide, ou les mélanges de cette dernière, et la substance solidifiante, ou les mélanges de cette dernière, sont mélangées selon un rapport de 95 + 5 à 80 + 20.

35. Dispersion de particules lipidiques selon l'une des revendications précédentes, **caractérisée en ce que** la phase aqueuse (agent de dispersion) contient un gélifiant pour épaississement, en particulier que la phase aqueuse est semi-solide sous l'effet de l'utilisation d'un gélifiant hydrophile, le seuil d'écoulement étant supérieur à 5 Pa à 21°C, et ce gélifiant étant choisi dans le groupe comprenant les alginates, les dérivés de la cellulose, la gomme xanthane, l'amidon, les bentonites et le monostéarate de glycérol, en particulier dans le groupe comprenant les polymères polyélectrolytiques, en particulier le poly(acide acrylique), la carboxyméthylcellulose ou le carraghénane.

36. Utilisation de dispersions de particules lipidiques selon l'une des revendications 8 à 18 en tant que liquide de granulation dans une opération de granulation pour fabriquer un granulé sec (en particulier pour le remplissage d'un sachet, ou dans des gélules), ou après compression d'un tel granulé pour la fabrication d'un comprimé.

37. Utilisation de dispersions de particules lipidiques selon l'une des revendications 8 à 18 en tant que liquide d'empâtement pour une masse d'extrusion pour la fabrication d'un produit sec, en particulier de pastilles.

38. Utilisation de dispersions de particules lipidiques selon l'une des revendications 8 à 18, **caractérisée en ce que** les dispersions sont transvasées dans des capsules en gélatine molle en utilisant une phase extérieure non aqueuse.

39. Utilisation de dispersions de particules lipidiques selon l'une des revendications 8 à 18 sous forme de pommade (en particulier d'une pommade pouvant être étalée) ou d'une lotion (en particulier sous forme d'une lotion visqueuse) pour application topique, la dispersion présentant une consistance suffisamment élevée à moyenne, qui éventuellement est encore augmentée par addition d'un gélifiant à la phase extérieure ou à la phase lipophile supplémentaire (en particulier de l'huile sous forme dispersée).

40. Utilisation de la dispersion de particules lipidiques selon l'une des revendications précédentes pour la stabilisation et/ou le ralentissement de la libération et/ou pour la diminution de l'effet irritant de substances actives essentiellement incluses dans les gouttelettes d'huile, en particulier dans le cas d'une administration orale, parentérale ou topique.

Abbildung 1

Abbildung 2

Abbildung 3

Schale, α-Modifikation

Feststoffbrücke

Abbildung 4

| ∿ Paraffin | ⎯o Fettalkohol |
| ⎓ Wasser | ⎯■ Fettalkoholsulfat |

Abbildung 5

Imwitor 1d

Imwitor 168d

Abbildung 6

Abbildung 7

Abbildung 8

1 Tag                     14 Tage

Abbildung 9

Abbildung 10

Abbildung 11

Abbildung 12

Abbildung 13

NCC (ca. 300 nm)

gesättigte
Wirkstofflösung

Lipidübergang
$\beta i/\beta$

übersättigte
Wirkstofflösung

Applikation

Haut

Nano-
compartiment
(flüssige Lipide)

$H_2O$

Diffusionsdruck

NCC-Gel im Gefäß
während Lagerung

EP 1 176 949 B1

Abbildung 14

Abbildung 15

Abbildung 16

Abbildung 17

2 Theta (°)

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19819273
- EP 0448930 A
- EP 0525307 A
- US 5188837 A
- US 5250236 A
- EP 0167825 A
- EP 0605497 A
- UA 5250236 A
- EP 0506197 A1
- DE 19707309 A1

### In der Beschreibung aufgeführte Nicht-Patentliteratur

- Feste Lipidnanopartikel. **MÜLLER, R.H.** Pharmazeutische Technologie: Moderne Arzneiformen. Wissenschaftliche Verlagsgesellschaft Stuttgart, 1998, 357-366
- **MOREL, S. ; UGAZIO, E. ; CAVALLI, R. ; GASCO, M.R.** Thymopentin in solid lipid nanoparticles. *Int. J. Pharm.,* 1996, 259-261
- **FREITAS, C. ; MÜLLER, R.H.** Effect of light and temperature on zeta potential and physical stability in Solid Lipid Nanoparticle (SLN™) Dispersions. *Int. J. Pharm.,* 1998, 221-229
- **LIST, P.H.** Arzneiformenlehre. Wissenschaftliche Verlagsgesellschaft Stuttgart, 1976, 264
- **MÜHLEN, A. ; SCHWARZ, C. ; MEHNERT, W.** Solid Lipid Nanoparticles (SLN) for controlled drug delivery - Drug release and release mechanism. *Eur. J. Pharm. Biopharm.,* 1998, vol. 45, 149
- **LUKOWSKI, G. ; WERNER, U. ; PFLEGEL, P.** surface investigation and drug release of drugloaded solid lipid nanoparticles. *Proc. 2nd World Meeting APGI/APV,* 1998, 573-574
- **FREITAS, C. ; MÜLLER, R.H.** Correlation between long-term stability of solid lipid nanoparticles (SLN™) and crystallinity of the lipid phase. *Eur. J. Pharm. Biopharm.,* 1999, vol. 47, 125-132
- **FREITAS, C. ; LUCKS, J.S. ; MÜLLER, R.H.** Effect of storage conditions on long-term stability of ''Solid Lipid Nanoparticles'' (SLN) in aqueous dispersion. *Proc. 1st World Meeting APGI/APV, Budapest,* 1995, 493-494
- **SUCKER, H. ; FUCHS, P. ; SPEISER, P.** Pharmazeutische Technologie. Georg Thieme Verlag Stuttgart, 1978
- **BAUER, K.H. ; FRÖMMING, K.-H. ; FÜHRER, C.** Pharmazeutische Technologie. G. Fischer Verlag Stuttgart, 1997, 276
- **J.M. ZEIDLER ; F. EMLING ; W. ZIMMERMANN ; H.J. ROTH.** *Archiv der Pharmazie,* 1991, vol. 324, 687
- **R. ZEISIG ; D. ARNDT ; H. BRACHWITZ.** *Pharmazie,* 1990, vol. 45, 809-818
- **K. THOMA ; P.SERNO ; D. PRECHT.** Röntgendiffraktometrischer Nachweis der Polymorphie von Hartfett. *Pharm. Ind,* 1983, vol. 45, 420-425
- Crystal structures of fats and fatty acids. **L. HERNQVIST.** Crystallisation and polymorphism of fats and fatty acids. Marcel Dekker Inc, 1988, 97-138
- **I. HASSAN.** *Phasenverhalten langkettiger Glyceride, Dissertation, Christian-Albrechts-Universität Kiel,* 1988
- Solidification and polymorphism in cacoa butter and the blooming problems. **J. SCHLICHTER-ARONHIME ; N. GARTI.** Crystallisation and polymorphism of fats and fatty acids. Marcel Dekker Inc, 1988, 363-392
- **H. YOSHINO ; M. KOBAYASHI ; M. SAMEJIMA.** Influence of fatty acid composition on the properties and polymorphic transition of fatty suppository bases. *Chem. Pharm. Bull.,* 1983, vol. 31, 237-246
- **WESTESEN, K. ; BUNJES, H. ; KOCH, M.H.J.** Physicochemical characterisation of lipid nanoparticles and evaluation of their drug loading capacity and sustained release potential. *J. Control. Release,* 1997, vol. 48, 223-236
- **ELDEM, T et al.** Optimization of spray dried and congealed lipid micropellets and characterisation of their surface morphology by scanning electron microscopy. *Pharm. Res.,* 1991, vol. 8, 47-51
- **TSUTSUMI et al.** *J. Soc. Cosmet. Chem.,* 1979, vol. 30, 345-356
- **WESTESEN, K. et al.** Physicochemical characterisation of lipid nanoparticles and evaluation of their drug loading capacity and sustained release potential. *J. Control. Rel.,* 1997, vol. 48, 223-236
- **CLERMONT-GALLERANDE, H.** *Feste kosmetische Zubereitung auf Basis verfestigter Öle,* 1998
- **BAUER et al.** Pharmazeutische Technologie. Georg Thieme Verlag, 1993, 43

- **BAUER et al.** Pharmazeutische Technologie. 1993, 253

- **RÜCKER et al.** Instrumentelle pharmazeutische Analytik. Wissenschaftliche Verlagsgesellschaft mbH Stuttgart, 1992, 172